(19) Europäisches Patentamt
European Patent Office
Office européen des brevets

(11) EP 2 348 031 B1

(12) # EUROPEAN PATENT SPECIFICATION

(45) Date of publication and mention of the grant of the patent:
**17.06.2015 Bulletin 2015/25**

(21) Application number: **10010646.7**

(22) Date of filing: **16.03.2001**

(51) Int Cl.:
*C07J 41/00* (2006.01) *A61K 31/57* (2006.01)
*C07J 31/00* (2006.01) *C07J 7/00* (2006.01)
*C07J 5/00* (2006.01) *C07J 9/00* (2006.01)
*A61K 31/575* (2006.01) *A61P 5/36* (2006.01)

(54) **17-Alpha-substituted-11-beta-substituted-4-aryl and 21-substituted 19-norpregnadienediones as antiprogestational agents**

17-Alpha-substituiertes-11-Beta-substituiertes-4-aryl und 21-substituiertes 19-Norpregnadiendionen als Antiprogestationswirkstoffe

17-alpha-substitué-11-bêta-substitué-4-aryle et 21-substitué 19-norpregnadienediones en tant que nouveaux agents antiprogestatifs

(84) Designated Contracting States:
**AT BE CH CY DE DK ES FI FR GB GR IE IT LI LU MC NL PT SE TR**

(30) Priority: **17.03.2000 US 526855**

(43) Date of publication of application:
**27.07.2011 Bulletin 2011/30**

(62) Document number(s) of the earlier application(s) in accordance with Art. 76 EPC:
**08009259.6 / 2 033 965**
**01918812.7 / 1 265 911**

(73) Proprietor: **The Government of the United States of America as represented by the Secretary of the Department of Health and Human Services**
**Rockville, MD 20852-3804 (US)**

(72) Inventors:
- **Kim, Hyun K.**
  **Bethesda, MD 20817 (US)**
- **Blye, Richard P.**
  **Highland, MD 20777 (US)**
- **Rao, Pemmaraju N.**
  **San Antonio, TX 78228 (US)**
- **Cessas, James W.**
  **Floresville, TX 78114 (US)**
- **Acosta, Carmie K.**
  **San Antonio, TX 78213 (US)**

(74) Representative: **Campbell, Patrick John Henry**
**J A Kemp**
**14 South Square**
**Gray's Inn**
**London WC1R 5JJ (GB)**

(56) References cited:
**WO-A-00/34306** **WO-A-89/12448**
**WO-A-97/41145**

- **RAO P N ET AL: "11beta-Substituted 13beta-ethyl gonane derivatives exhibit reversal of antiprogestational activity", STEROIDS: STRUCTURE, FUNCTION, AND REGULATION, US,ELSEVIER SCIENCE PUBLISHERS, NEW YORK, NY, vol. 63, no. 1, 1998, pages 50-57, XP004103060, ISSN: 0039-128X**
- **C. EDGAR COOK ET AL: "Effect of a 17[alpha]-(3-Hydroxypropyl)-17[beta]-acety l Substituent Pattern on the Glucocorticoid and Progestin Receptor Binding of 11[beta]-Arylestra-4,9-dien-3-ones", ORGANIC LETTERS, vol. 3, no. 7, 10 March 2001 (2001-03-10), pages 1013-1016, XP55009737, ISSN: 1523-7060, DOI: 10.1021/ol007067b**

EP 2 348 031 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The present invention relates generally to the field of steroids and, in particular, to novel 17-α-substituted, 11-β-substituted-4-aryl and 21-substituted 19-norpregnadienedione analogs which possess potent antiprogestational activity with minimal antiglucocorticoid activity.

### BACKGROUND OF THE INVENTION

There have been numerous attempts over the past few decades to prepare steroids with antibormonal activity. These have been reasonably successful where antiestrogens and antiandrogens are concerned. However, the discovery of effective antiprogestational and antiglucocorticoid steroids has proved to be a formidable task for the steroid chemist. It has been generally recognized for some years, however, that antiprogestational steroids would find wide applicability in population control, while antiglucocorticoids would be extremely valuable in the treatment of, for example, Cushing's syndrome and other conditions characterized by excessive endogenous production of cortisone. In the last decade, largely through the efforts of Teutsch, *et al.* of the Roussel-Uclaf group in France, a new series of 19-nortestosterone derivatives has been synthesized with strong affinity for the progesterone and glucocorticoid receptors and with marked antiprogestational and antiglucocorticoid activity *in vivo*. WO-A-89/12448 discloses anti-progestational and anti-glucocorticoid compounds which are 11.beta.-phenyl 19-norprogn-4,9-dien-3,20-dione derivative. This important discovery revealed the existence of a pocket in the progesterone/glucocorticoid receptors that is able to accommodate a large 11β-substituent on selected 19-nortestosterone derivatives. By suitable selection of such a substituent, steroids with antihormonal properties were obtained.

The pioneering studies of Teutsch, *et al.* on the synthesis of antiprogestational and antiglucocorticoid steroids is summarized in a recent review article (G. Teutsch in Adrenal Steroid Antagonism. Ed. M. K. Agarwal, Walter de Gruyter and Co., Berlin, 1984. pp. 43-75) describing the work leading to the discovery of RU-38,486, the first steroid of this type selected for clinical development. RU-38,486 or mifepristone was found to be an effective antiprogestational/contragestative agent when administered during the early stages of pregnancy (IPPF Medical Bulletin 20; No. 5, 1986). In addition to these antiprogestational properties, mifepristone has very significant antiglucocorticoid activity and was successfully used by Nieman, et al., J. Clin, Endocrinology Metab., 61:536, (1985)) in the treatment of Cushing's syndrome. In common with the vast majority of steroidal hormone analogs, mifepristone additionally exhibits a range of biological properties. Thus, for example, it exhibits growth-inhibitory properties towards estrogen-insensitive T47Dco human breast cancer cells (Horwitz, Endocrinology, 116:2236, 1985). Experimental evidence suggests that the metabolic products derived from mifepristone contribute to its antiprogestational and antiglucocorticoid properties (Heikinheimo, et al., J. Steroid Biochem., 26:279 (1987)).

Ideally, for purposes of contraception, it would be advantageous to have compounds which possess antiprogestational activity without (or with minimal) antiglucocorticoid activity. Although there have been a number of attempts to modify the mifepristone structure in order to obtain separation of the antiprogestational activity from the antiglucocorticoid activity, this goal has not yet been fully achieved. As such, there remains a need in the art for the development of new steroids which possess antiprogestational activity with minimal antiglucocorticoid activity.

### SUMMARY OF THE INVENTION

The present invention provides new steroids which possess potent antiprogestational activity with minimal antiglucocorticoid activity.

More particularly, the present invention provides compounds 65, 104b, 105b, 105c, 106b, 106c, 113d, 122b and 123b as disclosed in the examples and figures 4 and 7-9 and as specified in the claims. Further compounds are also disclosed herein, including compounds 73 and 112d.

As explained above, the compounds of the present invention possess potent antiprogestational activity with minimal antiglucocorticoid activity and, thus, they are suitable for long term use in the treatment of human endocrinologies or other conditions in steroid-sensitive tissues. Specific conditions for treatment include, but are not limited to, endometriosis (Kettel, L.M., et al., Fertil Steril, 56:402-407; Murphy, A.A., et al., Fertil Steril, 6:3761-766; Grow, D.R, et al., J. Clin. Endocrinol. Metab., 81:1933-1939:) uterine leiomyoma (Murphy, A.A., *et al., Ibid.;* Murphy, A.A., et al., J. Clin. Endocrinol. Metab., 76:513-517), uterine fibroid (Brogden, R.N., et al., Drugs, 45:384:409), meningioma (Brogden, RN., *et al., Ibid*.; Poisson, M., et al., J. Neurooncol., 1:179-189; Carroll, R.S., et al., Cancer Res., 53:1312-1316; Mahajan, D.K. and London, S.N., Fertil Steril, 68:967-976 (1997)), and metastatic breast cancer (Brogden, R.N., *et al., Id*.; Rochefort, H., Trends in Pharmacol. Sci., 8:126-128; Horwitz, K.B., Endocr. Rev., 13:146-163 (1992) Mahajan, D.K. and London. S.N., *Id*.). Other uses include, but are not limited to, contraception (Wood, A.J.J., N. engl. J. Med., 329:404-412 (1993);

Ulmann, A., et al., Sci. Amer., 262:42-48 (1990)), emergency postcoital contraceptive (Reel, J.R., et al., Contraception, 58:129-136 (1998)) and inducement of cervical ripening.

As such, in addition to providing the above mentioned compounds, the present invention provides these compounds for use in methods wherein the compounds are advantageously used, *inter alia,* to antagonize endogenous progesterone; to induce menses; to treat endometriosis; to treat dysmenorrhea; to treat endocrine hormone-dependent tumors (*e.g.,* breast cancer, uterine leiomyomas, *etc.*); to treat meningiomas; to treat uterine fibroids; to inhibit uterine endometrial proliferation; to induce cervical ripening; to induce labor; and for contraception.

Other features, objects and advantages of the invention and its preferred embodiments will become apparent from the detailed description which follows.

## BRIEF DESCRIPTION OF THE DRAWINGS

**Figures 1** through **11** illustrate the synthetic schemes used to prepare the compounds of the invention and related compounds not forming part of the present invention.

## DETAILED DESCRIPTION OF THE INVENTION AND PREFERRED EMBODIMENTS

The compounds of the present invention can readily be synthesized in a variety of ways using modern synthetic organic chemistry techniques. Typically, the compounds of the present invention are prepared using the synthetic schemes set forth in Figures 4 and 7-9. In general, there are five strategic steps that are useful in the synthesis of the antiprogestational agents of the present invention. They are: (1) C21-substitution; (2) construction of the 17α-hydroxy-20-ketone pregnane side chain with the natural configuration via the SNAP reaction; (3) modification of the 17α-hydroxy moiety; (4) regiospecific synthesis of the epoxide and 1,4-conjugate grignard addition of a variety of 4-substituted aryl compounds; and (5) deketalization at C3 and 20 and concomitant dehydratration at C5. Each of these five strategic steps is described in greater detail hereinbelow. Moreover, a more detailed description of the synthetic protocols used to prepare the compounds of the present invention is set forth in the Example Section. It will be readily apparent to those of skill in the art that the particular steps, or combination of steps, used will vary depending on the compound being synthesized. Some parts of the following description of the synthetic techniques, some of the examples and figures 1-3, 5, 6, 10 and 11 relate to the synthesis of related compounds other than those of the present invention, but which nonetheless illustrate synthetic techniques relevant to the synthesis of the compounds of the present invention.

Synthesis of the 17α,21-dimethoxy derivative (113d) was achieved via oxidation at C-21 to afford the 21-hydroxy derivative (107) of the 17a-methoxy compound (94) following a modification of the procedure reported by Moriarty, R.M. et al., J. Chem. Soc. chem. Commun., 641-642 (1981), *and* Velerio, et al., Steroids, 60:268-271 (1995). Subsequent O-methylation provided the key 17α,21-dimethoxy intermediate (**108**) (*see,* **Figure 8**). Reduction of the 20-ketone (**108**) to the 20ξ-ol (**109**) followed by epoxidation at C5 and C10, copper (I) catalyzed conjugate Grignard addition to the 5α, 10α-epoxide (**110**), selective oxidation of the secondary alcohol, 20ξ-ol (**111**) using IBX to the 20-ketone (**112**), hydrolysis and acetylation, led to the target 17α,21-dimethoxy derivative (113d).

### 2. Silicon Nucleophilic Annelation Process (SNAP)

As described herein silylation of β-cyanohydrin ketal with halomethyldimethylsilyl chloride afforded the chloro- or broroomethyl dimethylsilyl ether. The reductive SNAP reaction provided the 17α-hydroxy-20-ketopregnane side chain with the natural configuration at C17 (Livingston, D.A., et al., J. Am. Chem. Soc., 112:6449-6450 (1990); Livingston, D.A., Adv.Med. Chem., 1:137-174 (1992); U.S. Patent No. 4,092,693, which issued to Livingston, D.A., et al. (May 1, 1990); U.S. Patent No. 4,977,255, which issued to Livingston, D.A., et al. (December 11, 1990). Alternatively, the formation of the halomethyldimethylsilyl ether, followed by treatment with lithium diisopropyl amide, provided the 21-substituted -17α-hydroxy-20-ketopregnanes,

### 3. 17α-Substitution

All 17α-esters illustrated in Figures 4-11 were prepared from their 17α-hydroxy precursors. With the exception of the 17α-formate (**69A**) and the 17a,21-diformate (139), all 17α-esters were also obtained via a mixed anhydride procedure (Carruthers, N.I. et al., J. Org. Chem., 57:961-965 (1992)).

17α-methoxy steroid (93) became available in large quantities from the 17α-hydroxydienedione (**92**) leading to a new series of antiprogestational agents, such as compounds 97 and 113d. Methylation of 17α-hydroxy group was most efficiently carried out using methyl iodide and silver oxide with acetonitrile as a cosolvent as described in the general procedure of Finch, et al. (J. Org. Chem., 40:206-215 (1975)). Other syntheses of 17α-methoxy steroids have been reported in the literature (*see, e.g.,* Numazawa, M. and Nagaoka, M., J. Chem. Soc. Commun., 127-128 (1983); Numazawa, M. and Nagaoka, M., J. Org. Chem., 50:81-84 (1985); Glazier, E.R., J. Org. Chem., 27:4397-4393 (1962).

**4. 11β-Aryl-4-Substution**

The introduction of a variety of 4-substituted phenyl group at C11β into 19-norprogesterone requires the 5α,10α-epoxide. Epoxidation of **2, 23, 34, 42, 50, 88, 94, 99, 109** and **119** has been known to be problematic (*see,* Wiechert, R. and Neef, G., J. Steroid Biochem., 27:851-858 (1987)). The procedure developed by Teutsch, G., *et al.* (Adrenal Steroid Antagonism (Agarwal, M.K., ed.), 43-75, Walter de Gruyter & Co., Berlin, N.Y. (1984)), *i.e.,* $H_2O_2$ and hexachloro or fluoroacetone, proved to be regioselective, but not highly stereoselective. A mixture of 5α,10α-epoxide and the corresponding 5β,10β-isomer was formed in approximately a 3:1 ratio. However, reduction of the C20-ketone (**108**) to the C20-ol (**109**) prior to opoxidation, resulted in a 9:1 ratio of the desired 5α,10α-epoxide.

Treatment of the 5α,10α-epoxides with 3-5 equivalents of Grignard reagents prepared from various 4-substituted aryl bromides *(see,* Yux'ev, Y.K., et al,, Izvest. Akad. Nauk. S.S.S.R., Otdel Khim Nauk, 166-171 (CA 45: 10236f, (1951)); Wolfe, J.P. and Buchwald, S.L., J. Org. Chem., 62:6066-6068 (1997); Veradro, G., et al., Synthesis, 447-450 (1991); Jones, D.H., J. Chem. Soc. (C), 132-137 (1971); Detty et al., J. Am. Chem. Soc., 105:875-882 (1983), and Rao, P.N. et al., Steroids, 63:523-550 (1998)) in the presence of copper (1) chloride as a catalyst provided the desired 11β-4-substituted phenyl steroids. It is noted that 4-bromothioanisole was purchased from the Aldrich Chemical Co. (Milwaukee, Wisconsin). Evidence of the 11β-orientation of the 4-substituted phenyl substituent was shown by the upfiled shift of the C18 methyl group ($\delta$ = 0.273 0.484 ppm in $CDCl_3$), which is in agreement with Teutsch's observations (*see,* Teutsch, G. and Belanger, A., Tetrahedron Lett., 2051-2054 (1979)).

The presence of an unprotected 20-ketone resulted in low yields or in undesirable Grignard product mixtures. This was circumvented by reduction of the 20-ketone (analysis of this material by NMR indicated a single isomer; no further work was done for identification of this single isomer) prior to epoxidation and subsequent oxidation of the 20-alcohol by use of iodoxybenzoic acid (IBX) (Dess, D.B. and Martin, J.C., J. Org. Chem., 48:4155-4156 (1983); Frigerio, M. and Santagostino, M., Tetrahedron Letters, 35:8019-8022 (1994); and Frigerio, M, et al., J. Org. Chem., 60:7272-7276) after Grignard addition (*see,* **Figure 8**).

5. Deketalization

Deketalization with concomitant dehydration at C-5 in acidic media proceeded smoothly to provide the 4,9-dione-3,20-dione.

Quite surprisingly, the compounds of the invention possess potent antiprogestational activity with minimal antigincocorticoid activity. As a result of their antiprogestational activity, the compounds of the invention can advantageously be used, *inter alia,* to antagonize endogenous progesterone; to induce menses; to treat endometriosis; to treat dysmenorrhea; to treat endocrine hormone-dependent tumors; to treat meningioma; to treat uterine leiomyonas, to treat uterine fibroids; to inhibit uterine endometrial proliferation; to induce labor; to induce cervical ripening, for hormone therapy; and for contraception.

More particularly, compounds having antiprogestational activity are characterized by antagonizing the effects of progesterone. As such, the compounds of the present invention are of particular value in the control of hormonal irregularities in the menstrual cycle, for controlling endometriosis and dysmenonhea, and for inducing menses. In addition, the compounds of the present invention can be used as a method of providing hormone therapy either alone or in combination with astrogenic substances in postmenopausal women, or in women whose ovarian hormone production is otherwise compromised.

Moreover, the compounds of the present invention can be used for control of fertility during the whole of the reproductive cycle. For long-term contraception, the compounds of the present invention can be administered either continuously or periodically depending on the dose. In addition, the compounds of the present invention are of particular value as postcoital contraceptives, for rendering the uterus inimical to implantation, and as "once a month" contraceptive agents.

A further important utility for the compounds of the present invention lies in their ability to slow down growth of hormone-dependent tumors and/or tumors present in hormone-responsive tissues. Such tumors include, but are not limited to, kidney, breast, endometrial, ovarian, and prostate tumors, *e.g.,* cancers, which are characterized by possessing progesterone receptors and can be expected to respond to the compounds of this invention. In addition, such tumors include meningiomas. Other utilities of the compounds of the present invention include the treatment of fibrocystic disease of the breast and uterine.

Compounds suitable for use in the above method of the present invention can readily be identified using *in vitro* and *in vivo* screening assays known to and used by those of skill in the art. For instance, a given compound can readily be screened for its antiprogestational properties using, for example, the anti-McGinty test and/or the anti-Clauberg

test described in the examples. In addition, a given compound can readily be screened for its ability to bind to the progesterone and/or glucocorticoid receptors or to inhibit ovulation using the assays described in the examples. Moreover, a given compound can readily be screened for its ability to inhibit tumor cell growth (*e.g.,* malignant tumor growth, *i.e.,* cancer) or to abolish tumorigenicity of malignant cells *in vitro* or *in vivo.* For instance, tumor cell lines can be exposed to varying concentrations of a compound of interest, and the viability of the cells can be measured at set time points using, for example, the alamar Blue® assay (commercially available from BioSource, International of Camarillo, California). Other assays known to and used by those of skill in the art can be employed to identify compounds useful in the methods of the present invention.

The compounds according to the present invention can be administered to any warm-blooded mammal such as humans, domestic pets, and farm animals. Domestic pets include dogs, cats, *etc.* Farm animals include cows, horses, pigs, sheep goats, *etc.*

The amount of active ingredient that can be combined with a carrier material to produce a single dosage form will vary depending upon the disease treated, the mammalian species, and the particular mode of administration. For example, a unit dose of the steroid can preferably contain between 0.1 milligram and 1 gram of the active ingredient. A more preferred unit dose is between 0.001 and 0.5 grams. It will be understood, however, that the specific dose level for any particular patient will depend on a variety of factors including the activity of the specific compound employed; the age, body weight, general health, sex and diet of the individual being treated; the time and route of administration; the rate of excretion; other drugs which have previously been administered; and the severity of the particular disease undergoing therapy, as is well understood by those of skill in the area.

The compounds of the present invention can be administered by a variety of methods. Thus, those products of the invention that are active by the oral route can be administered in solutions, suspensions, emulsions, tablets, including sublingual and intrabuccal tablets, soft gelatin capsules, including solutions used in soft gelatin capsules, aqueous or oil suspensions, emulsions, pills, lozenges, troches, tablets, syrups or elixirs and the like. Products of the invention active on parenteral administration can be administered by depot injection, implants including Silastic TM and biodegradable implants, intramuscular and intravenous injections.

Compositions can be prepared according to any method known to the art for the manufacture of pharmaceutical compositions and such compositions can contain one or more agents selected from the group consisting of sweetening agents, flavoring agents, coloring agents and preserving agents. Tablets containing the active ingredient in admixture with nontoxic pharmaceutically acceptable excipients which are suitable for manufacture of tablets are acceptable. These excipients can be, for example, inert diluents, such as calcium carbonate, sodium carbonate, lactose, calcium phosphate or sodium phosphate, granulating and disintegrating agents, such as maize starch, or alginic acid; binding agents, such as starch, gelatin or acacia; and lubricating agents, such as magnesium stearate, stearic acid and talc. Tablets can be uncoated or, alternatively, they can be coated by known methods to delay disintegration and adsorption in the gastrointestinal tract and thereby provide a sustained action over a longer period. For example, a time delay such as glyceryl monostearate or glyceryl distearate alone or with a wax can be employed.

Formulations for oral use can also be presented as hard gelatin capsules wherein the active ingredient is mixed with an inert solid diluent, for example calcium carbonate, calcium phosphate or kaolin, or as soft gelatin capsules wherein the active ingredient is mixed with water or an oil medium, such as peanut oil, liquid paraffin or olive oil.

Aqueous suspensions of the invention contain the active materials in admixture with excipients suitable for the manufacture of aqueous suspensions. Such excipients include a suspending agent, such as sodium carboxymethylcellulose, methylcellulose, hydroxypropylmethylcellulose, sodium alginate, polyvinylpyrolidone, gum tragacanth and gum acacia, and dispersing or wetting agents such as a naturally occurring phosphatide (*e.g.,* lecithin), a condensation product of an alkylene oxide with a fatty acid (*e.g.,* polyoxyethylene stearate), a condensation product of ethylene oxide with a long chain aliphatic alcohol (*e.g.,* heptadecaethylene oxycetanol), a condensation product of ethylene oxide with a partial ester derived from a fatty acid and a hexitol (*e.g.,* polyoxyethylene sorbitol mono-oleate), or a condensation product of ethylene oxide with a partial ester derived from fatty acid and a hexitol anhydride (*e.g.,* polyoxyethylene sorbitan monooleate). The aqueous suspension can also contain one or more preservatives such as ethyl or n-propyl p-hydroxybenzoate, one or more coloring agents, one or more flavoring agents and one or more sweetening agents, such as sucrose, aspartame or saccharin. Ophthalmic formulations, as is known in the art, will be adjusted for osmolarity.

Oil suspensions can be formulated by suspending the active ingredient in a vegetable oil, such as arachis oil, olive oil, sesame oil or coconut oil, or in a mineral oil such as liquid paraffin. The oil suspensions can contain a thickening agent, such as beeswax, hard paraffin or cetyl alcohol. Sweetening agents can be added to provide a palatable oral preparation. These compositions can be preserved by the addition of an antioxidant such as ascorbic acid.

Dispersible powders and granules of the invention suitable for preparation of an aqueous suspension by the addition of water can be formulated from the active ingredients in admixture with a dispersing, suspending and/or wetting agent, and one or more preservatives. Suitable dispersing or wetting agents and suspending agents are exemplified by those disclosed above. Additional excipients, for example sweetening, flavoring and coloring agents, can also be present.

The pharmaceutical compositions of the invention can also be in the form of oil-in-water emulsions. The oily

phase can be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan monooleate, and condensation products of these partial esters with ethylene oxide, such as polyoxyethylene sorbitan monooleate. The emulsion can also contain sweetening and flavoring agents.

Syrups and elixirs can be formulated with sweetening agents, such as glycerol, sorbitol or sucrose. Such formulations can also contain a demulcent, a preservative, a flavoring or a coloring agent

The pharmaceutical compositions of the invention can be in the form of a sterile injectable preparation, such as a sterile injectable aqueous or oleaginous suspension. This suspension can be formulated according to the known art using those suitable dispersing or wetting agents and suspending agents which have been mentioned above. The sterile injectable preparation can also be a sterile injectable solution or suspension in a nontoxic parenterally-acceptable diluent or solvent, such as a solution of 1,3-butanediol. Among the acceptable vehicles and solvents that can be employed are water and Ringer's solution, an isotonic sodium chloride. In addition, sterile fixed oils can conventionally be employed as a solvent or suspending medium. For this purpose any bland fixed oil can be employed including synthetic mono- or diglycerides. In addition, fatty acids such as oleic acid can likewise be used in the preparation of injectables.

The compounds of this invention can also be administered in the form of suppositories for rectal administration of the drug. These compositions can be prepared by mixing the drug with a suitable non-irritating excipient which is solid at ordinary temperatures but liquid at the rectal temperatures and will therefore melt in the rectum to release the drug. Such materials are cocoa butter and polyethylene glycols.

They can also be administered by in intranasal, intraocular, intravaginal, and intrarectal routes including suppositories, insufflation, powders and aerosol formulations.

Products of the invention which are preferably administered by the topical route can be administered as applicator sticks, solutions, suspensions, emulsions, gels, creams, ointments, pastes, jellies, paints, powders, and aerosols.

The invention will be described in greater detail by way of specific examples. The following examples are offered for illustrative purposes, and are intended neither to limit or define the invention in any manner.

EXAMPLES

EXAMPLE 1

This example illustrates the preparation and properties of 17α-Acetoxy-11β-(4-acetylphenyl)-19-norpregna-4,9-diene-3,20-dione(73) (**Figure 4**).

*Step 1. 3,20-bis-Ethylenedixoy-17α-hydroxy-19-norpregna-5(10),9(11)-diene (50):*

A mixture of 17α-hydroxy-19-norpregna-4,9-diene-3,20-dione (**92**,10 g, 31.8 mmol), ethylene glycol (11.10 g, 178.7 mmol), freshly distilled triethyl orthoformate (14g, 94.1 mmol) and toluenesulfonio acid monohydrate (0.3 g, 1.58 mmol) in $CH_2Cl_2$ (150 mL) was stirred at room temperature under nitrogen overnight. Analysis by TLC (5% acetone in $CH_2Cl_2$) at that time indicated a complete reaction. Solid $NaHCO_3$ (~1 g) was added and the mixture was diluted with $CH_2Cl_2$ (~100 mL) and poured into $H_2O$. The mixture was extracted with $CH_2Cl_2$ (3x). The organic fractions were washed with $H_2O$ (3x), filtered through sodium sulfate, combined and concentrated *in vacuo* to give 12 g of the crude product 50 as a yellow foam, Crystallization ofthis crude material from $CH_2Cl_2$/MeOH containing a trace of pyridine gave 9.8 g of the pure diketal 50 as a light yellow solid in 77% yield; m.p. 169 - 171°C. FTIR(KBr, diffuse reflectance) $\nu_{max}$ 3484 and 2912 $cm^{-1}$. NMR (300 MHz, $CDCl_3$): δ 0.792 (s, 3 H, C18-$CH_3$), 1.378 (s, 3 H, C21-$CH_3$), 3,816 and 4.047 (m, 4H, C20-ketal), 3.983 (s, 4H, C3-ketal) and 5.555 (m, 1 H, C11-CH=). MS (EI) m/z (relative intensity): 402 ($M^+$, 100.0), 366 (2.5), 340 (20.8) 270 (59.9) and 99 (50.1).

*Step 2. 3,20-bis-Ethylenedtoxy-17α-hydroxy-5α,10α-epoxy-19-norpregna-9(11)-ene (51):*

Hydrogen peroxide (30%, 3.3 mL, 32.31 mmol) was added to a solution of hexafluoroacetone trihydrate (3.34 g, 16.17 mmol) in $CH_2Cl_2$ (53 mL) cooled to 0°C. Solid $Na_2HPO_4$ (1.48 g, 10.43 mmol) was added and the mixture stirred at 0°C for ½ hr. A solution of the 3,20-diketal (50, 6.0 g, 14.9 mmol) in $CH_2Cl_2$ (45 mL), precooled to 0°C, was added over a period of 10 min and the reaction mixture was stirred overnight at 5°C. Analysis by TLC (5% acetone in $CH_2Cl_2$) at that point indicated absence of the starting material. The reaction mixture was diluted with $CH_2Cl_2$ (~100 mL) and washed with 10% $Na_2SO_3$ solution (2x) and saturated $NaHCO_3$ solution (2x). The organic fractions were filtered through $Na_2SO_4$, combined and concentrated *in vacuo* to give 7 g of 51 of a white foam. Trituration of the epoxide mixture (a and β) with ether afforded 3.05 g of the pure 5α,10α-epoxide 51 as a white solid in 48.9% yield; m.p. = 172-173°C. FTIR (KBr, diffuse reflectanoe) $\nu_{max}$ 3439, 2950, 1705, 1642 and 1593 $cm^{-1}$. NMR (300 MHz, $CDCl_3$) δ 0.789 (s, 3 H, C18-

$CH_3$), 1.365 (s, 3 H, C21-$CH_3$), 3.810 - 4.094 (m, 8 H, C3- and C20-ketals) and 6.013 (m, 1 H, C11-CH=). MS (EI) m/z (relative intensity): 418 ($M^+$, 0.5), 400 (1.4), 293 (0.9), 131 (2.5), 99 (4.3) and 87 (100.00).

*Step 3 3,20-bis-Ethylenedioxy-5α,17α-dihydroxy-11β-[4-(2-methyl-1,3-dioxolan-2-yl)phenyl]-19-norpregn-9-ene (56):*

Magnesium turnings (435 mg, 17.9 mmol) were weighed into a 100 mL round bottom two-neck flask equipped with a reflux condenser, a magnetic stirrer and a rubber septum. A small crystal of iodine was added and the system was flushed with dry nitrogen and flame dried. After the system had cooled to room temperature, freshly distilled THF (20 mL) was introduced *via* syringe followed by a small amount of dry dibromoethane (~0.1 mL). After evidence of reaction was observed (disappearance of $I_2$ color, bubble formation on metal), a solution of the ketal of 4-bromoacetophenone (*see,* Detty, M.R., et al., J. Am. Chem. Soc., 105:875-882 (1983); and Rao, P.N., et al., Steroids, 63:523-530 (1998)) (4.35 g, 17.9 mmol) in dry THF (10 mL) was added via syringe. The mixture was then stirred in a hot water bath for 2 hr. (After 35 min, an additional 10 mL of THF was added as a white precipitate formed and the reaction mixture thickened). The reaction was cooled to room temperature and copper (I) chloride (177 mg, 1.79 mmol) was added and the mixutre stirred at room temperature for ½ hr (the precipitate went back into solution with the addition of the copper chloride). The 5α,10α-epoxide (51, 1.5 g, 3.58 mmol) in dry THF (10 mL) was added via syringe and the reaction mixture stirred at room temperature for 45 min. At this time, TLC (10% acetone in $CH_2Cl_2$) showed no starting material. Saturated $NH_4Cl$ solution (~20 mL) was added and the mixture stirred at room temperature for ½ hr while air was drawn through the reaction mixture to oxidize the copper. The contents of the flask were diluted with $H_2O$ (~100 mL) and extracted with $CH_2Cl_2$ (3x). The organic fractions were washed with saturated $NH_4Cl$ solution (1x), $H_2O$ (1x), and brine (1x), and then dried over anhydrous $Na_2SO_4$, filtered and concentrated *in vacuo* to yield an oil. The oil was purified on a flash column (10% acetone in $CH_2Cl_2$) yielding 1.3 g of a stable white foam. The material was crystallized from ether to yield 880 mg of 56 as a white crystalline solid in 42.3% yield; m.p. = 185-188°C. FTIR (KBr, diffuse reflectance) $\nu_{max}$ 3501, 2940, 1609, 1443, 1371, 1181 and 1042 $cm^{-1}$. NMR ($CDCl_3$) δ 0.45 (s, 3 H, C18-$CH_3$), 1.4 (s, 3 H, $CH_3$ from ethylene ketal of acetophenone at C11β), 1.6 (s, 3 H, C21-$CH_3$), 3.6 - 4.2 (br m, 12 H, C3- and C20-ketals and ketal of acetophenone at C11β-), 4.3 (br d, 1H, C11α-CH), and 7.05 - 7.47 (dd, 4 H, aromatic-CH's). MS(EI) m/z (relative intensity): 582 ($M^+$). Anal, Calcd. for $C_{34}H_{46}O_8$; C, 70.08; H, 7.96. Found: C, 70.00; H, 8.05.

*Step 4 17α-Hydroxy-11β-(4-Acetylphenyl)-19-norpregna-4,9-diene-3,20-dione (65):*

Nitrogen was bubbled through a mixture of EtOH (25 mL) and 8.5% $H_2SO_4$ (2:5 mL) for ½ hr to remove oxygen. The Grignard adduct (57, 750 mg, 1.28 mmol) was added as a solid with stirring. The mixture was put into an oil bath preheated to 95°C for 1 hr. The mixture was cooled in an ice bath and quenched with saturated $K_2CO_3$ to bring the pH to ~10. The mixture was diluted with $H_2O$ (125 mL) and extracted with $CH_2Cl_2$ (3x). The organic fractions were washed with saturated $NaHCO_3$ (1x), $H_2O$ (1x), brine (1x), combined and dried over anhydrous $Na_2SO_4$. This material was concentrated *in vacuo* to give 600 mg of 65 as an oil. Tho material was purified on a flash column (10% acetone in $CH_2Cl_2$) to yield 560 mg of 65. This material was crystallized from $CH_2Cl_2$ and ether to give 475 mg of 65 as a white solid in 85.9% yield; m.p. = foams/honeycombs at 112-115°C. FTIR (KBr, diffuse reflectance) $\nu_{max}$ 3390, 2976, 1709, 1679, 1655, 1601, 1360 and 1275 $cm^{-1}$. NMR ($CDCl_3$) δ 0.4 (s, 3 H, C18-$CH_3$), 2.25(s, 3 H, C21-$CH_3$), 2.6 (s, 3 H, 11β-4-phenylacetyl $CH_3$), 3.25 (s, 1H, C17α-OH),, 4.5 (br d, 1 H, C11α-CH), 5.8 (s, 1 H, C4-CH=) and 7.2 - 8.0 (dd, 4 H, aromatic-CH's), MS (EI) m/z (relative intensity): 432($M^+$, 88.7), 414 (11,3), 389 (25.4), 371 (21.1), 346(100:0), 331 (46.5), 319 (22.5), 280 (15.5), 235 (16.9), 200 (14.1), 147(18.3), 133 (18.3), 115 (12.7), 105 (15.5) and 91 (21.1)

*Step 5. Preparation of the target compound 73:*

The triketone (65, 375 mg, 0.87 mmol) was dissolved in $CH_2Cl_2$ (10 mL) and cooled to 0°C in an ice bath. In a separate roundbottom flask, trifluoroacetic anhydride (3.65 g, 17.3 mmol) and acetic acid (1.14 g, 17.3 mmol) were added to $CH_2Cl_2$ (10 mL), flushed with dry nitrogen and stirred at room temperature for ½ hr. The mixed anhydride was then placed in an ice bath and cooled to 0°C. The cold mixed anhydride solution was then added to the triketone (65) solution and treated with *p*-toluenesulfonic acid (152 mg, 0.79 mmol). The reaction mixture was stirred for 45 min at 0°C. The reaction was quenched with saturated $K_2CO_3$ (pH = 10), diluted with $H_2O$ and extracted with $CH_2Cl_2$ (3x). The organic layers were combined, washed with $H_2O$ (2x), brine (1x), dried over sodium sulfate, filtered and concentrated to yield 425 mg of crude 73. The crude product 73 was purified on a flash column (10% acetone in $CH_2Cl_2$) to yield 340 mg of compound 73. Crystallization from $CH_2Cl_2$, and ether afforded 305 mg of 73 as white solid in 73.96% yield; m.p. = 243-246°C.

Analysis by reverse phase HPLC on a Waters Nova Pak $C_{18}$ column eluted with MeOH:$H_2O$ in the ratio of 70:30 at a flow rate of 1 mL/min and at λ = 260 nm indicated it to be 99.6% pure. FTIR (KBr, diffuse reflectance) $\nu_{max}$ 2791, 1729, 1712, 1681, 1595, 1362, and 1257 $cm^{-1}$, NMR ($CDCl_3$) δ 0.3 (s, 3 H, C18-Me), 2.10 (s, 3 H, C17α-OAc),

2.15 (s, 3 H, C21-$CH_3$), 2.55 (s, 3 H, 11β-4-phenylacetyl $CH_3$), 4.5 (br d, 1 H, C11α-CH), 5.8 (s, 1 H, C4-CH=) and 7.2 - 8.0 (dd, 4 H, aromatic-CH's). MS (EI) m/z (relative intensity): 474($M^+$, 2.8), 414 (36.6), 399 (14.0), 389 (8.5) and 371 (100). Anal. Calcd. for $C_{30}H_{34}O_5$ ½$Et_2O$: C, 74.85; H, 7.44, Found: C, 74.94; H, 7.19.

EXAMPLE 2

This example illustrates the preparation and properties of 17α,21-Diacetoxy-11β-(4-acetylphenyl)-19-norpregna-4,9-diene-3,20-dione(106b) (Figure 7);

*Step 1. 3,3-Ethylenedioxy-17β-cyano-17α-trimethylsilyloxyesttra-5-(10),9(11)-diene (99);*

Under nitrogen, pyridine (136.9 g, 1740 mmol) solution of the cyanohydrin ketal (98, 25 g, 73.22 mmol) was treated with chlorotrimethylsilane (44g, 394 mmol). The mixture was stirred at room temperature overnight. The reaction mixture was poured into a 50:50 mixture of ice/saturated $NaHCO_3$ solution (~1.2 L), stirred until the ice had melted, and extracted with hexane (3x). The organic extracts were washed with $H_2O$ (3x), brine (1x), combined, dried over anhydrous $Na_2SO_4$, and concentrated *in vacuo.* The remaining pyridine was azeotropically removed *in vacuo* with heptane. Crystallisation of the residue from pentane gave 26.1 g of the pure silyl ether (99) as a white solid in 86.2% yield; m.p, = 99 - 101°C, FTIR (KBr, diffuse reflectance) $\nu_{max}$ 2944, 2908, 2231 and 1253 $cm^{-1}$. NMR (300 MHz, $CDCl_3$) δ 0.229 (s, 9 H, C17α-OSi($CH_3$)$_3$), 0.894 (s, 3 H, C18-$CH_3$), 3.987 (s, 4 H, 3-O$CH_2CH_2$O) and 5.615 (t, 1 H, J = 2.55 Hz, C11-CH=). MS (EI) m/z (relative intensity): 413 ($M^+$, 100.0), 398 (5.5), 385 (24.0), 371 (6.4), 237 (33.9) and 69.3 (86.0).

*Step 2. 3,3-Ethylenedioxy-5α-10α-epoxy-17β-cyano-17α-trimethylsilyloxyestr-9(11)-ene (100):*

Hydrogen peroxide (30%, 12 mL), 117.12 mmol) was added to a vigorously stirred mixture of hexafluoroacetone trihydrate (20.20 g, 112.5 mmol) in $CH_2Cl_2$ (185 mL) cooled to 0°C in an ice bath. The reaction mixture was stirred at 0°C for ½ hr, and solid $Na_2HPO_4$ (11g, 77.5 mmol) was added followed by an ice-cold solution of the silyl ether (99,25 g, 60.44 mmol) in $CH_2Cl_2$ (185 mL). The mixture was then stirred at 0°C for 5 hr, then at 5°C overnight. Analysis by TLC (5% acetone in $CH_2Cl_2$) at that time indicated a complete reaction. The reaction mixture was diluted with $CH_2Cl_2$ (~200 mL) and washed with 10% $Na_2SO_3$ solution (1x), $H_2O$ (1x) and brine (1x). The organic fractions were filtered through anhydrous sodium sulfate, combined and concentrated *in vacuo.* Trituration of the residue with ether afforded 16.66 g of the pure 5α,10α-epoxide (100) as a white solid in 64.16% yield; m.p. 156 - 160°C. FTIR (KBr, diffuse reflectance) $\nu_{max}$ 2955, and 2228 $cm^{-1}$, NMR (300 MHz, $CDCl_3$) δ 0.219 (s, 9 H, OSi($CH_3$)$_3$), 0.894 (s, 3 H, C18-$CH_3$), 3.85 - 3.97 (s, 4 H, C3-O$CH_2CH_2$O) and 6.082 (t, 1 H, J = 2,6 Hz, C11-CH=). MS(EI) ml (relative intensity): 429 ($M^+$, 18.5), 401(2.8), 343 (11.1), 238 (9.5), 99 (100.0) and 86 (36.2).

*step 3. 3,3-Ethylenedioxy-5α-hydroxy-11β-[4-(2-methyl-1,3-dioxolan-2-yl)phenyl]-17β-cyano-17α-trimethylsilyloxyestr-9-ene (101b):*

Under nitrogen and in flame-dried glassware, dry THF (240 mL) was added to magnesium turnings (2.3 g, 94.6 mmol). Solid bromoacetophenone ketal (*see,* EXAMPLE 1 Step 3) (20.79 g, 85.5 mmol) was added and the mixture heated to refux. After ½ hr of reflux, evidence of Grignard formation such as cloudiness and color change was observed. Heating was discontinued and tho mixture stirred for 1 hr, after which time most of the magnesium had reacted and a substantial amount of the precipitated Grignard ragent was observed. Solid CuCl (4 g, 40.4 mmol) was added and the mixture was stirred at room temperature for 15 min, after which time the solid reagent went back into solution. A solution of the 5α,10α-epoxide (100, 17.5 g, 40.73 mmol) in THF (150 mL) was added and the reaction mixture was stirred at room temperature for 1 hr. After that time, TLC(5% acetone in $CH_2Cl_2$) of a small aliqout quenched with saturated $NH_4Cl$ solution indicated a complete reaction. The reaction was quenched by the addition of saturated $NH_4Cl$ solution (~50 mL). In order to oxidize Cu(I) to Cu(II), air was drawn through the reaction mixture for ½ hr. The resulting blue mixture was diluted with ether (500 mL) and washed with $H_2O$ (2x), brine (1x), dried over anhydrous $Na_2SO_4$, filtered and concentrated *in vacuo* to give 41 g of the residue as an oil. Crystallization of this crude material from ether gave the pure 101b (23.0 g) as a white solid in 95% yield; m.p. = 192 - 193°C. FTIR (KBr, diffuse reflectance): $v_{max}$ 3515, 2951, 2884, 2230, 1619, 1505 and 1102 $cm^{-1}$. NMR ($CDCl_3$): δ 0.25 (s, 9 H, Si($CH_3$)$_3$), 0.5 (s, 3 H, C18-$CH_3$), 1.67 (s, 3 H, C11β-(acetophenone ketal $CH_3$), 3.67 - 4.17 (m, 8 H, C3- O$CH_2CH_2$O-), 4.37 (m, 2 H, C11α-CH plus OH), 7.17 (d, 2 H, J = 9 Hz, 2', 6' aromatic-CH's) and 7.37 (d, 2 H, J = 9 Hz, 3', 5' aromatic-CH's). MS (EI) m/z (relative intensity): 593 ($M^+$, 3.6), 578 (6.0), 575 (9.1), 560 (2.5), 366 (5.2) 99 (27.3) and 87 (100.0). Anal. Calcd. for $C_{3A}H_{47}NO_6Si$: C, 68.77; H, 7.98; N, 2.36. Found: C, 68.69; H, 7.87; N, 2.43.

*Step 4. 17β-cyano-17α-hydroxy-11β-(4-acetylphenyl)-estra-4,9-dien-3-one (102b);*

A solution of the Grignard adduct (101b, 23 g, 38.7 mmol) was dissolved in THF (100 mL) and the system was flushed with nitrogen. Glacial acetic acid (314.7 g, 524 mmol) and $H_2O$ (100 mL) were added and the mixture was stirred overnight at room temperature. At that time, TLC (10% acetone/$CH_2Cl_2$) indicated an incomplete reaction. The reaction mixture was then heated to reflux for 1 hr, after which time TLC indicated a complete reaction.

The volatiles were removed *in vacuo* at 50°C and the residue diluted with $H_2O$ (~250 mL) and saturated $NaHCO_3$ solution (~125 mL). The subsequent precipitate was extracted with EtOAc (5x) with some difficulty in that the crude product was relatively insoluble in most solvents used. The organic fractions were washed with $H_2O$ (2x), brine (1x), combined, dried over anhydrous sodium sulfate, filtered and concentrated *in vacuo*. Trituration of the residue with ether gave the cyanohydrin (102b, 16.3 g) as a light yellow solid in 100% yield; m.p. = 141 - 143°C (dec), FTIR (KBr, diffuse reflectance): $\nu_{max}$ 3362, 2966, 2946, 2232, 1619, 1730, 1658 and 1600 $cm^{-1}$. NMR ($CDCl_3$ + $d_6$ DMSO): δ 0.57 (s, 3 H, C18-$CH_3$), 2.60 (s, 3 H, C11β-(4-phenyl-C(O)$CH_3$), 4.57 (br s, 1 H, C11α-CH), 5.80 (s, 1 H, C4-CH=), 7.40 (d, 2 H, J = 9 Hz, 2', 6' aromatic-CH's) and 7.97 (d, 2 H, J = 9 Hz, 3', 5' aromatic-CH's), MS(EI) m/z (relative intensity): 415 ($M^+$,0.5), 404 (0.4), 388 (100.0), 292 (65) and 97 (51.0). Anal. Calcd. for $C_{27}H_{29}NO_3 \cdot 1/3H_2O$: C, 76.93; H, 7.09; N, 3.32. Found: C, 77.04; H, 6.99; N, 3.45.

*Step 5. 11β-(4-acetylphenyl)-17β-cyano-17α-bromethyldimethylsilyloxyestra-4,9-dien-3-one (103b):*

Under nitrogen, a solution of the cyanohydrin (102b, 15 g, 36.12 mmol), $Et_3N$ (6.53 g, 64 mmol) and DMAP (2.6 g, 21.3 mmol) in dry THF (180 mL) was treated with bromomethyldimethylsilyl chlorine (9.70 g, 54 mmol). The mixture was stirred overnight at room temperature, diluted with ether (500 mL), filtered through Celite and *concentrated in vacuo.* The relative insolubility of this material (103b) precludes chromatographic purification useing ether as eluent. The crude material (103b) was used directly in the subsequent reaction without further purification or characterization.

*Step 6. 17α-Hydroxy-11β-(4-acetylphenyl)-21-bromo-19-norpregna-4,9-dien-3-one (104b);*

Under anhydrous conditions and using a mechanical stirrer, a solution of the silyl ether (103b) (assumed 20.34 g, 36.12 mmol) in dry THF (500 mL) was cooled to - 78°C and treated dropwise with a 1.5 M solution of lithium diisopropylamide (LDA) in cyclohexane (100 mL, 150 mmol). After 1 hr, the reaction mixture became very viscous, almost a gel. The reaction was quenched at -78°C by addition of 4.45 M HBr (500 mL, 890 mmol) and the mixture allowed to warm to room temperature. After stirring at room temperature for 1 hr, the excess acid was neutralized by slow addition of concentrated $NH_4OH$ solution (~60 mL), The mixture was further diluted with $H_2O$ (~200 mL) and extracted with $CH_2Cl_2$ (3x). The organic fractions were washed with $H_2O$ (3x), combined, filtered through $Na_2SO_4$ and concentrated *in vacuo* to give 20 g of the residue as a foam. This material was purified *via* flash chromatography eluted with (10% acetone in $CH_2Cl_2$) to give 2.6 g of the 21-bromo product (104b) as a white solid in 14.1% yield. FTIR (KBr, diffuse reflectance): $\nu_{max}$ 3340, 2946, 1723, 1693, 1679, 1645 and 1601 $cm^{-1}$. NMR ($CDCl_3$): δ 0.33 (s, 3 H, C18-$CH_3$), 2.19 (s, 3 H, 11β-(4-phenyl-C(O)$CH_3$), 4.30 - 4.70 (m, 3 H, C11α-CH and C21-$CH_2$Br), 5.83 (s, 1 H, C4-CH=), 7.33 (d, 2 H, J = 9 Hz, 2', 6' aromatic-CH's) and 7.93 (d, 2 H, J = 9 Hz, 3',5' aromatic-CH's). MS (EI) m/z (relative intensity): 512 ($M^+$,24.1), 466 (100), 432 (48.5), 431 (48.5), 430 (86,4), 371 (71,9) and 91 (76.0).

*Step 7. 17α-Hydroxy-11β-(4-acetylphenyl)-21-acetoxy-19-norpregna-4,9-diene-3,20-dione (105b):*

A mixture of the 21-bromo drivative (104b, 2.5 g, 4.89 mmol), anhydrous KOAc (20 g, 203.8 mmol) in dry $CH_3CN$ (100 mL) was heated to reflux under nitrogen, After 2 hr, TLC (10% acetone in $CH_2Cl_2$) indicated a complete reaction. The reaction mixture was cooled to room temperature, filtered and concentrated *in vacuo* to give 2.6 g as a foam. This material was purified via flash chromatography (12% acetone in $CH_2Cl_2$) followed by cyrstallization from EtOAc to give 1.5 g of the pure 17α-ol-21-acetate (105b) as a light yellow solid in 62.6% yield; m.p. = softens at 110°C, FTIR (KBr, diffuse reflectance): $\nu_{max}$ 3467, 2948, 1749, 1727, 1727, 1681, 1380, 1664 and 1603 $cm^{-1}$. NMR ($CDCl_3$): δ 0.31 (s, 3 H, C18-$CH_3$), 2.15 (s, 3 H, C17α-OC(O)$CH_3$), 2.57 (s, 3 H, 11β-4-phenyl-C(O)C$\underline{H}_3$), 4.5 (br d, 1 H, C11α-CH), 5.01 (dd, 2 H, $J_1$= 18.7 Hz, $J_2$ = 18 Hz, C21-$\underline{CH_2}$OAc), 5.81 (s, 1 H, C4-CH=), 7.34 (d, 1 H, J = 8.2 Hz, 2', 6' aromatic-CH's), 7.35 (d, 1 H, J = 6.8 Hz, 2', 6' aromatic-CH's) and 7.93 (d, 2 H, J = 8.2 Hz, 3', 5' aromatic-CH's), MS (EI) m/z (relative intensity): 490 ($M^+$, 88.0), 430 (100.0), 344 (80.0), 236 (44.0), and 91 (55.0). Anal, Calcd. for $C_{30}H_{34}O_6 \cdot 1/5CH_2Cl_2$ C, 70.99; H, 6.79, Found: C, 70.83; H, *6.65.*

*Step 8._Preparation of the target compound 106b:*

Under nitrogen trifluoroacetic anhydride (11.15 g, 53.2 mmol), glacial acetic aoid (3025 g, 54.2 mmol) in dry

$CH_2Cl_2$ (35 mL) were combined and stirred at room temperature for ½ hr. *p*-Toluenesulfonic acid monohydrate (0.5 g, 2.63 mmol) was added and the reaction mixture was cooled to 0°C in an ioe bath. A solution of the 17α-ol-21-acetate (105b, 1.28 g, 2.61 mmol) in dry $CH_2Cl_2$ (10 mL) was precooled to 0°C and then added. The reaction mixture was stirred at 0°C. After 45 min, TLC (10% acetone in $CH_2Cl_2$) indicated a complete reaction. The mixture was quenched at 0°C with concentrated $NH_4OH$ solution (~10 mL, ~148 mmol), allowed to warm to room temperature, and diluted with $H_2O$ (~50 mL). The pH of the aqueous fraction was adjusted to 5 with concentrated $NH_4OH$ solution and the mixture extracted with $CH_2Cl_2$ (3x). The organic fractions were washed with $H_2O$ (3x), combined, dried over anhydrous $Na_2SO_4$, filtered and concentrated *in vacuo* to give 1.8 of the crude product as a foam. The crude material was purified via flash chromatography (5% acetone in $CH_2Cl_2$) to give 1.1 g of the purified diacetate (106b) as a foam. Crystallization of this foam from EtOAc / heptane afforded 0.78 g of the pure solid (106b) as a white crystalline solid in 56.1% yield.; m.p. = 197 - 199°C, Reverse phase HPLC analysis on Phonomenex Prodigy 5 ODS-2 column eluted with $H_2O/CH_3CN$, 1: 1 at a flow rate of 1 mL/min and at λ = 302 nm indicated this material to be >99% pure with a retention time ($t_R$) of 5.6 min. FTIR (KBr, diffuse reflectance): $v_{max}$ 2951, 1757, 1678, 1664 and 1604 $cm^{-1}$. NMR ($CDCl_3$): δ 0.33 (s, 3 H, C18-$CH_3$), 2.07 (s, 3 H, C17α-OC(O)$CH_3$), 2.10 (s, 3 H, C21-OAc), 2.50 (s, 3 H, C11β-4-phenyl-C(O)$CH_3$), 4.43 (m, 1 H, C11α-CH), 4.77 (dd, 2 H, $J_1$= 32.9 Hz, $J_2$ = 14.9 Hz, C21-$CH_2$OAc), 5.77 (s, 1 H, C4-CH=), 7.23 (d, 2 H, J = 8 Hz, 2', 6' aromatic-CH's), and 7.83 (d, 2H, J = 8 Hz, 3', 5' aromatic-CH's). MS (EI) m/z (relative intensity): 532 ($M^+$, 6.2), 472 (17.3), 412 (11.3), 371 (100.0) and 91 (14.3). Anal. Calcd. for $C_{32}H_{36}O_7 \cdot 1/7H_2O$: C, 71.81; H, 6.83. Found: C, 71.89; H, 6.87.

EXAMPLE 3

This example illustrates the preparation and properties of 17α-Acetoxy-11β-(4-acetylphenyl)-21-thioacetoxy-19-norpregna-4,9-diene-3,20-dione (106c) (Figure 7):

*Step 1. 17α-Hydroxy-11β-(4-acetylphenyl)-21-thioacetoxy-19-norpregna-4,9-diene-3,20-dione (105c):*

A mixture of the 21-bromo derivative (104b, 5.746 g, 11.23 mmol), sodium iodide (16.84 g, 112.3 mmol), potassium thioacetate (12.83 g, 112.3 mmol) in dry acetone (600 mL) was heated to reflux under nitrogen. After 4 hr, TLC (50% EtOAc in hexanes) indicated a complete reaction. The reaction was cooled to room temperature, filtered, concentrated *in vacuo,* diluted with $H_2O$ (~200 mL) and extracted with $CH_2Cl_2$ (3x). The organic fractions were washed with $H_2O$ (1x) and brine (1x), combined, dried over anhydrous sodium sulfate, concentrated *in vacuo* to give the crude product as a yellow foam. This material was purified by flash chromatography (50% EtOAc in hexanes) followed by crystallization from EtOAc/hexanes to afford the pure 17α-ol-21-thioacetate (105c, 3.25 g, 57.1%) as a white crystalline solid; m.p. = 159 - 160°C. FTIR (KBr, diffuse reflectance): $v_{max}$ 3325, 2950, 1723, 1688, 1637 and 1590 $cm^{-1}$. NMR ($CDCl_3$): δ 0.33 (s, 3 H, C18-$CH_3$), 2.4 (s, 3 H, C21-SC(O)$CH_3$), 2.57 (s, 3 H, C11β-4-phenyl-C(O)$CH_3$), 4.0 (dd, 2 H, $J_1$ = 48.6 Hz, $J_2$ = 18 Hz, C21- $CH_2$SAc), 4.57 (br d, 1 H, C11α-CH), 5.8 (s, 1 H, C4-CH=), 7.37 (d, 2 H, J = 9 Hz, 2', 6' aromatic-CH's), and 7.93 (d, 2 H, J = 9 Hz, 3', 5' aromatic-CH's). MS(EI) m/z (relative intensity): 506 ($M^+$, 29.1), 488 (14.4), 474 (16.6), 431 (100.0) and 346 (78.1). Anal. Calcd. for $C_{30}H_{34}O_5S \cdot H_2O$: C, 68.68; H, 6.92; S, 6.11. Found: C,68,99; H, 6.73; S, 6.06.

***Step 2. Preparation of the target compound 106c:***

Under nitrogen, trifluoroacetic anhydride (17.43 g, 82.89 mmol), glacial acetic acid (7.17 g) 118.45 mmol), *p*-toluenesulfonic acidmonohydrate (1.0 g, 5.3 mmol) and dry $CH_2Cl_2$ (100 mL) were combined and stirred at room temperature for ½h. The mixture was cooled to 0°C in an ice bath and a solution of the 17α-ol-21-thioacetate (105e, 3.0 g, 5.92 mmol) in dry $CH_2Cl_2$ (54 mL) was added. The mixture was stirred at 0°C for 6 hr after which time TLC (4% acetone/$CH_2Cl_2$) indicated a complete reaction. The mixture was neutralized with cold saturated $NaHCO_3$ and extracted with $CH_2Cl_2$, (3x), The organic fractions were washed with brine (2x), combined, dried over sodium sulfate and concentrated *in vacuo* to give the crude product as a foam. Purification of this material by Flash chromatography eluting 4% acetone/$CH_2Cl_2$ followed by crystallization from EtOAc/hexanes gave 2.34 g of the pure compound 106c as a yellow crystalline solid; m.p. = 204 - 205°C. FTIR (KBr, diffuse reflectance): $v_{max}$ 2948, 1734, 1702, 1676, 1663 and 1602 $cm^{-1}$. NMR($CDCl_3$): δ 0.30 (s, 3 H, C18-$CH_3$), 2.15 (s, 3 H, C17α-OC(O)$CH_3$), 2.33 (s, 3 H, C21-SC(O)$CH_3$), 2.57 (s 3 H, C11β-4-phenyl-C(O)$CH_3$), 3.94 (dd, 2H, $J_1$= 20.7 Hz, $J_2$ = 14.4 Hz, C21-$CH_2$SAc), 4.53 (br d, 1H, C11α-CH), 5.83 (s, 1 H, C4-CH=), 7.37 (d; 2 H, J = 9 Hz, 2', 6' aromatic-CH's), and 7.93 (d, 2 H, J = 9 Hz, 3', 5' aromatic-CH's), MS (EI) m/z (relative intensity): 548 ($M^+$, 6.9), 488 (18.4), 413 (27.4), 371 (100.0) and 280 (24.0). Anal. Calcd. for $C_{32}H_{36}O_6S \cdot 1/10H_2O$: C, 69.82; H, 6.63; S, 5.82. Found; C, 68.83; H, 6.67, S, 5.59.

**EXAMPLE 4**

This example illustrates the preparation and properties of 17α,21-Dimethoxy-11β-(4-acetylphenyl)-19-norpregna-4,9-diene-3,20-dione (113d) (**Figure 8**):

***Step 1. 3,3-Ethylenedioxy-17α-methoxy-21-hydroxy-19-norpregna-5(10),9(11)-dien-20-one (107):***

To a solution of the 17α-methoxy-3-ketal (**94**, 10.0 g, 27.1 mmol) in dry THF (150 mL) was added iodobenzene diacetate (Moriarty, et al., J. Chem. Soc., Chem. Commun., 641-642 (1981); Velerio, et al., Steroids, 60:268-271 (1995)) (34.59 g, 4x) as a solid. The suspension was stirred under nitrogen and cooled to 0°C. $H_2O$ (7.73 mL, 429.6 mmol, 16x) was added, followed by 0.5 M KO-tBu solution (1400 mL, 700 mmol, 26x) via transfer needle. (A 50:50 (v/v) mixture of freshly opened methanol (700 mL) and 1.0 M potassium t-butoxide in THF (700 mL; Aldrich) was prepared and cooled to 0°C to give a 0.5 M base solution). Upon completion of addition the reaction mixture was removed from the ice bath and the solution alowed to warm to room temperature. The reaction was monitored every hour by TLC (5% acetone in $CH_2Cl_2$) and after 4 hr, virtually all of the starting material had been converted to approximately a 80:20 mixture of two more polar components. The reaction mixture was diluted with $H_2O$ (500 mL) and brine (500 mL) and extracted into ether (3x). Organic fractions were washed again with $H_2O$ and brine. Combined organic extracts were dried by filtration through $Na_2SO_4$, evaporated *in vacuo,* and further dried under high vacuum to recover 13.84 g of an orange oil. Purification by flash chromatography (5% acetone in $CH_2Cl_2$) gave 6.0 g of a pale yellow-white foam (107) in 57.5% yield. Trituration with pentane produced 107 which was dried under vacuum to recover 5.36 g of a white powder in 51.0% yield; m.p. = 147 - 152°C. FTTR (KBr, diffuse reflectance): $v_{max}$ 3478, 2900, 2825, 1712,1437, 1384 and 1372 $cm^{-1}$. NMR (300 MHz, $CDCl_3$): δ 0.550 (s, 3 H, C18-$CH_3$), 3.159 (s, 3 H, C17α-$OCH_3$), 3.981 (s, 4 H, C3-$O\underline{CH_2CH_2}O$), 4.251 and 4.471 (AB, 2 H, $J_{AB}$ = 19.81 Hz, C21-$CH_2$) and 5.544 (br s, 1 H, C11-CH=). MS (EI) m/z (relative intensity): 388 ($M^+$, 54.8), 356 (13.8), 297 (100.0), 211 (65.0), 169 (51.1) and 99 (56.3). Anal. Calcd. for $C_{23}H_{32}O_5 \cdot 1/4H_2O$: C, 70.29; H, 8.34. Found: C, 70.21; H, 8.12.

***Step 2. 3,3-Ethylenedioxy-17α,21-dimethoxy-19-norpregna-5(10),9(11)-dien-20-one (108):***

To a solution of the 3-ketal-21-hydroxy compound (107, 5.0 g, 12.87 mmol) in 500 mL of 1,2-dimethoxyethane (DME) was added Proton-Sponge® [1,8-bis(dimethylamino)naphthalene] (13.79 g, 64.35 mmol, 5x) as a solid. The solution was cooled to 0°C in an ice water bath and trimethyloxonium tetrafluoroborate (9.52 g, 64.35 mmol, 5x) was added as a solid. The suspension was kept at 0°C under nitrogen, for 3 hr. At that time, TLC (5% acetone in $CH_2Cl_2$) indicated all of the starting material had been cleanly converted to the slightly less polar 3-ketal-17α,21-dimethoxy compound (108). $H_2O$ and EtOAc were added, the mixture was transferred to a separatory funnel, and the layers allowed to separate. The organic fraction was washed with ice-cold 1 N HCl (2x), $H_2O$ (1x), saturated $NaHCO_3$ (1x), $H_2O$ (1x), and brine (1x). Combind EtOAc extracts (3x) were dried by filtration through $Na_2SO_4$ and evaporated *in vacuo.* The resulting colorless oil was dried overnight under high vacuum to recover a white foam (108, 5.28 g) in quantitative yield. Analysis by TLC and NMR indicated the crude material was sufficiently pure to carry directly on to the next reaction. A small amount was triturated with pentane and dried overnight under high vacuum to give 120 mg of 108 as a white solid; m.p = 104-110°C. FTIR (KBr, diffuse reflectance): $v_{max}$ 2926; 2884, 2828,1722, 1447, 1380, 1322 and 1252 $cm^{-1}$. NMR (300 MHz, $CDCl_3$): δ 0.585 (s, 3 H, C18-$CH_3$), 3.175 (s, 3 H, C17α-$OCH_3$), 3.442 (s, 3 H, C21-$OCH_3$), 3.983 (s, 4 H, C3-$O\underline{CH_2CH_2}O$), 4.182 and 4.367 (AB, 2 H, $J_{AB}$ = 18.01 Hz, C21-$CH_2$) and 5.555 (br s, 1 H, C11-CH=). MS (EI) m/z (relative intensity): 402 ($M^+$, 27.7), 370 (7.2), 297 (100.0), 211 (62.1), 169 (41.6) and 99 (62.7). Anal. Calcd. for $C_{24}H_{34}O_5 \cdot 3/5H_2O$: C, 69.74; H, 8.58. Found: C, 69.82; H, 8.43.

***Step 3. 3,3-Ethylenedioxy-17α,21-dimethoxy-19-norpregna-5(10),9(11)-dien-20-ol (109):***

The 3-ketal 17α,21-dimethoxy-20-one (108, 5.0 g, 12.42 mmol) was dissolved in dry THF (100 mL) and 2 equivalents of $LiAlH_4$ (25 mL, 25 mmol, 1.0 M in ether) were added *via* syringe. The solution was stirred magnetically at room temperature under nitrogen. After 15 minutes, examination by TLC (5% acetone in $CH_2Cl_2$) indicated the starting material had been cleanly converted to a single, more polar product (109). The reaction mixture was cooled in an ice bath, and saturated $Na_2SO_4$ (~2 - 3 mL) was added dropwise *via* pipette. When the reaction was quenched, several scoops of $Na_2SO_4$ were added and the mixture allowed to stir 1 hr. Filtration through a sintered glass funnel, followed by evaporation *in vacuo* produced a concentrated syrup. The syrup was taken up in $H_2O$ and $CH_2Cl_2$, transferred to a separatory funnel, and the layers allowed to separate. The organic fraction was washed again with brine. Combined $CH_2Cl_2$ extracts (3x) were dried by filtration through $Na_2SO_4$ and evaporated *in vacuo.* The resulting white foam was dried further under high vacuum to recover 4.69 g of the crude 109. Purification of this crude product by flash chromatography (5% isopropanol in $CH_2Cl_2$) gave 4.24 g of 109 as a white foam in 84.4% yield.

The two purest fractions were combined and taken up in a minimum amount of acetone/hexane. After standing six days at room temperature, large, colorless crystals had formed. The crystals were collected by centrifugation, washed with several portions of hexane, and dried under high vacuum to recover 177 mg. Analysis by TLC (10% acetone in $CH_2Cl_2$) indicated the crystals were of the highest purity. Analysis of this material by NMR indicated a single isomer. No further work was done for identification of this single isomer. A second crop of 78 mg with only a trace of impurity was obtained from the mother liquors; m.p. =111- 115°C. FTIR (KBr, diffuse reflectance): $v_{max}$ 3576, 3456, 2930, 2891, 2827, 1460 and 1372 $cm^{-1}$. NMR (300 MHz, $CDCl_3$): δ 0.824 (s, 3 H, C18-$CH_3$), 3.298 (s, 3 H, C17α-$OCH_3$), 3.392 (s, 3 H, C21-$OCH_3$), 3.416 (dd, 1 H, $J_1$ = 9.30 Hz, $J_2$ = 8.10 Hz, C21-$CH_2$), 3,490 (dd, 1 H, J $_1$ = 9.30 Hz, J $_2$ = 3.30 Hz, C21-$CH_2$), 3.923 (dd, 1 H, $J_1$ = 8.10 Hz, $J_2$ = 3.30 Hz, C20-CH), 3.980 (s, 4 H, C3-O$\underline{CH_2CH_2}$O) and 5.595 (br s, 1 H, C11-CH=). MS (EI) m/z (relative intensity): 404 ($M^+$, 2.1), 372 (5.7), 329 (1.7), 297 (100.0) and 211 (35.7). Anal. Calcd. for $C_{24}H_{36}O_5 \cdot 1/5C_6H_{14}$: C, 71.76; H, 9.27. Found: C, 71.83; H, 9.04.

***Step 4. 3,3-Ethylenedioxy-5α,10α-epoxy-17α,21-dimethoxy-19-norpregn-9(11)-en-20-ol (<u>110</u>):***

To a solution of hexafluoroacetone (2.01 mL, 14.39 mmol) in $CH_2Cl_2$ (50 mL), was added solid $Na_2HPO_4$ (1.36 g, 9.59 mmol) and 30% $H_2O_2$ (2.16 mL, 21.1 mmol). The mixture was transferred to the cold room and stirred vigorously for % hr at 4°C. A chilled solution of the 20-alcohol (<u>109</u>, 3.88 g. 9.59 mmol) in $CH_2Cl_2$ (25 mL) was added via pipette and rinsed in with additional CH $2Cl_2$ (25 mL). After stirring overnight at 4°C, TLC (7.5% acetone in $CH_2Cl_2$) indicated virtually all of the starting material had been converted to one major, more polar product with only a trace of byproducts. The reaction mixture was transferred to a separatory funnel and washed with 10% $Na_2SO_3$ (1x), $H_2O$ (1x), and brine (1x). Combined CH $_2Cl_2$ extracts (3x) were dried by filtration through $Na_2SO_4$ and evaporated *in vacuo* to recover a foam. NMR analysis of the crude material indicated the a and β epoxides were present in approximately a 9:1 ratio. Trituration with ether produced 2.27 g of the pure 5α,10 a epoxide (<u>110</u>) as a white powder in 56.3% yield; m.p. =146 -153°C. FTIR (KBr, diffuse reflectance): $v_{max}$ 3558, 2939, 1638,1446,1373 and 1247 $cm^{-1}$. NMR (300 MHz, $CDCl_3$): δ 0.824 (s, 3 H, C18-$CH_3$), 3.273 (s, 3 H, C17α-$OCH_3$), 3.389 (s, 3 H, C21-$OCH_3$), 3.402 (dd, 1 H, $J_1$ = 9.61 Hz, $J_2$ = 8.10 Hz, C21 -$CH_2$), 3,476 (dd, 1 H, $J_1$ =9.1 Hz, $J_2$ =3.30Hz, C21-$CH_2$), 3.908 (m, 5 H, C3-O$\underline{CH_2CH_2}$O and C20-CH) and 6.053 (brs, 1 H, C11-CH=). MS (EI) m/z (relative intensity): 420 ($M^+$, 1.7), 402 (6.0), 370 (6.2), 345 (20.0), 313 (77.8), 295 (100.0) and 99 (95.4). Anal. Calcd. for $C_{24}H_{36}O_5 \cdot 1/10H_2O$: C, 68.25; H, 8.64. Found: C, 68.31; H, 8.71.

*Step 5: 3,3-Ethylenedioxy-5α-hydroxy-11β-[4-(2-methyl-1,3-dioxolan-2-yl)phéyl]-17α,21-dimethoxy-19-norpregn-9-en-20-ol (<u>111d</u>):*

Magnesium turnings (289 mg, 11.89 mmol) were weighed into a 100 mL round bottom two-neck flask equipped with a reflux condenser, a magnetic stirrer, and a rubber septum, A small crystall of iodine was added and the system was flushed with nitrogen and flame dried, After cooling to room temperature, freshly distilled THF (10 mL) was introduced *via* syringe followed by a small amount of dry dibromoethane (~0.1 mL). After evidence of reaction was observed (disappearance of $I_2$ color, and bubble formation on metal), a solution of the ketal of 4-bromoacetophenone (*see*, Example 20, Step 1) (2.89 g, 11.89 mmol) in dry THF (10 mL) was added via syringe. The mixture was then stirred in a hot water bath for 2 hr until the majority of the magnesium was consumed. After the reaction mixture was cooled to room temperature, solid copper (I) chloride (11.8 mg, 1.19 mmol) was added and the mixture was stirred at room temperature for ½ hr. The epoxide (<u>110</u>, 1.0 g, 2.38 mmol) in dry THF (10 mL) was added via syringe. The reaction mixture was stirred at room temperature for 1 hr then quenched with the addition of saturated $NH_4Cl$ solution (~20 mL), and the mixture was stirred at room temperature for ½ hr while air was drawn through the reaction mixture to oxidize Cu(I) to Cu(II). The contents of the flask were diluted with water (~100 mL) and extracted with $CH_2Cl_2$ (3x). The organic extracts were washed with saturated $NH_4Cl$ solution (1x), water (1x) and brine (1x), then dried over anhydrous sodium sulfate, filtered and concentrated *in vacuo* to yield 4.3 g of oil. This was purified on a flash column (10% acetone in $CH_2Cl_2$) to yield 850 mg of <u>111d</u> as a white foam which was triturated with ether to produce a white crystalline solid in 61.2% yield; m.p. = 145 - 150°C (Material changed to amber gel) and gel melts at 173 - 177°C. FTIR (KBr, diffuse reflectance): $v_{max}$ 3461, 2946, 2877, 2812, 1663, 1602, 1540, 1505, 1457 and 1372 $cm^{-1}$. NMR (300 MHz, $CDCl_3$): δ 0.443 (s, 3H, C18-$CH_3$), 1.636 (s, 3 H, $CH_3$ of acetophenone ketal), 3.289 (s, 3 H, C17α-$OCH_3$), 3.358 (s, 3 H, C21-$OCH_3$), 3.741-4.015 (m, 8 H, C3- and C11β-4-acetyl ketals), 4.244 (br s, 1 H, C11α-CH), 7.165-7.327 (dd, 4 H, aromatio-CH's). MS (EI) m/z (relative intensity): 584 ($M^+$). Anal. Calcd. for $C_{34}H_{48}O_8$: C, 69.86; H, 8.22. Found: C, 69.63; H, 8.28.

*Step 6: 3,3-Ethylenedioxy-5α-hydroxy-11β-[4-(2-methyl-1,3-dioxolan-2-yl)phenyl]-17α,21-dimethoxy-19-norpregn-9-en-20-one (112d):*

Under nitrogen, IBX (1.149 g, 4.104 mmol) was dissolved in DMSO (8 mL) over a period of 10 min. A solution of the Grignard product (<u>111d</u>, 800 mg, 1.368 mmol) in DMSO (8 mL) was added *via* pipette to the above solution and

the reaction mixture stirred at room temperature for ½ hr. At that time, TLC (10% acetone in $CH_2Cl_2$; aliquot was diluted in water and extracted into EtOAo) showed the starting material had been converted to a single less polar product. The reaction was diluted with $H_2O$ (~150 mL) and extracted with $CH_2Cl_2$ (3x). The organic layers were washed with $H_2O$ (1x) and brine (1x), dried over anhydrous sodium sulfate, filtered and concentrated in vacuo to give 820 mg of 112d as an off-white foam. This was purified on a flash column (10% acetone in $CH_2Cl_2$). The product was originally obtained as a foam and was triturated with pentane and dried *in vacuo* to yiled 540 mg of 112d as a white solid in 73% yield; m.p. = 102 -106°C (shrinkage to an amber gel); 111 - 113°C (gel bubbles); 123 - 133°C (gel melts). FTIR (KBr, diffuse reflectance): $v_{max}$ 3526, 2939, 2884, 2825, 1722, 1665 and 1604 $cm^{-1}$. NMR (300 MHz, $CDCl_3$): δ 0.190 (s, 3 H, C18-$CH_3$), 1.625 (s, 3 H, $CH_3$ of acetophenone ketal), 3.146 (s, 3 H, C17α-$OCH_3$), 3.445 (s, 3 H, C21-$OCH_3$), 3.742 and 4.015 (m, C3 and C11β-4-acetylphenyl ketals), 4.310 (d, 1 H, C11α-CH), 7.119 - 7.332 (dd, 4 H, aromatic-CH's) MS (EI) m/z (relative intensity): 582 ($M^+$), Anal. Calcd. for $C_{34}H_{46}O_8$: C, 70.08; H, 7.96 Found: C, 70.11; H, 8.01. FTIR (KBr, diffuse reflectance); $v_{max}$ 3526, 2939, 2884, 2825, 1722, 1665 and 1604 $cm^{-1}$. NMR (300 MHz, $CDCl_3$); δ 6.190 (s, 3H, C18-$CH_3$), 1.625 (s, 3 H, $CH_3$ of acetophenone ketal), 3.416 (s, 3 H, C17α-4$CH_3$), 3.445 (s, 3 H, C21-$OCH_3$), 3,742 and 4,015 (m, C3 and C11β-4-acetylphenyl ketals), 4.310 (d, 1 H, C11α-CH), 7.119-7.332 (dd, 4 H, aromatic-CH). MS (EI) m/z (relative intensity): 582 ($M^+$). Anal. Calcd. for $C_{34}H_{46}O_8$: C, 70.08; H, 7.96 Found: C, 70.11; H, 8.01.

***Step 7. Preparation of the target compound 113d:***

Nitrogen was bubbled through a mixture of EtOH (925 mL) and 8.5% sulfuric acid for ½ hr to remove oxygen. The 20-ketone (112d, 520 mg, 0.892 mmol) was added as a solid with stirring to the above solution. The mixture was put into an oil bath preheated to 95 °C and was refluxed under nitrogen for 1 hr. The reaction mixture was cooled in an ice bath and quenched with saturated $K_2CO_3$ solution (pH ≅ 10), diluted with water (~125 mL) and extracted with $CH_2Cl_2$ (3x). The organic fractions were washed with water and brine, dried over anhydrous $Na_2SO_4$, filtered and concentrated *in vacuo* to give 460 mg of the crude product, Flash chromatography (10% acetone in $CH_2Cl_2$) gave 377 mg of an off-white pale yellow solid. This was crystallized from mixture of distilled ether and $CH_2Cl_2$ to yield 360 mg of 113d in 81% yield as a white crystalline solid in two batches. The product 113d retained $CH_2Cl_2$ and required extreme drying: m.p. = 133-136°C (foams) and 172-178°C (foam melts). FTIR (KBr, diffuse reflectance): $v_{max}$ 2942, 1719, 1681, 1665, 1600, 1409, 1359 and 1272 $cm^{-1}$. NMR (300 MHz, $CDCl_3$): δ 0.264 (s, 3 H, C18-$CH_3$), 2.571 (s, 3 H, $CH_3$ of acetophenone ketal), 3.185 (s, 3 H, C17α-$OCH_3$), 3.449 (s, 3 H, C21-$OCH_3$), 4.183 and 4.385 (dd, 2 H, C21-$CH_2$-), 4.456 and 4.481 (d, 1 H, C11α-CH), 5.90 (s, 1 H, C4-CH=), 7.247 - 7.7883 (dd, 4H, aromatic-CH's). MS (EI) m/z (relative intensity): 476 ($M^+$, 35), 403 (93), 371 (100), 331 (67) and 91 (26). Anal. Calcd. for $C_{30}H_{36}O_5$: C, 75.63; H, 7.56. Found: C, 74.78; H, 7.58.

EXAMPLE 5

This example illustrates the preparation and properties of 17α-Acetoxy-11β-(4-acetylphenyl)-21-methoxy-19-norpregna-4,9-diene-3,20-dione (123b):

*Step 1. 17α-Hydroxy-21-chloro-19-norpregna-4,9-diene-3,20-dione (115):*

The 3-ketal cyanohydrin (98, 50g, 73.22 mmol) was magnetically stirred with freshly distilled THF (550 mL) under nitrogen at room temperature. 4-Dimethylaminopyridine (DMAP) (4.47 g, 36.59 mmol) was added as a solid. Freshly distilled $Et_3N$ (27.60 mL, 197.68 mmol) followed by freshly distilled chloro-(chloromethyl)dimethylsilane (25.1 mL, 190.36 mmol) was added *via* syringe. The reaction was allowed to stir overnight at room temperature. The next day TLC on silica (2% acetone in $CH_2Cl_2$) showed all starting material had been converted to the silyl ether. The reaction mixture was cooled to -78°C in a dry ice bath with isopropanol, and then diluted with THF (800 mL). Lithium diisopropylamide (LDA) (2.0 M, 300 mL, 600 mmol) was added dropwise to the reaction *via* an additional funnel over a period of 45 min. Once addition was complete, the reaction was stirred for 1.5 hr at -78°C, HCl (4 N, 1250 mL, 5 mol) was added *via* the addition funnel. The dry ice bath was removed, and the reaction was allowed to stir overnight at room temperature. The reaction mixture was then cooled to 0°C and neutralized by the addition of concentrated $NH_4OH$ (305 mL). The mixture was transferred to a separatory funnel and extracted with EtOAc (3x), washed with $H_2O$ (2x) and brine (1x). The organic fractions were combined, filtered through $Na_2SO_4$ and evaporated *in vacuo.* The resulting solid was triturated with ether (1000 mL), collected on a Buchner funnel, and washed with additional ether. After drying overnight *in vacuo,* 38.90 g of 115 as a dark yellow solid was recovered in 76.61% yield. Analysis by TLC on silica (5% acetone in $CH_2Cl_2$) showed the material was suitable to carry directly on to the next reaction; m.p. = 204 - 207°C. FTIR (KBr, diffuse reflectance): $v_{max}$ 3465, 2946, 1729, 1664, 1599 and 1573 $cm^{-1}$. NMR (300 MHz, $CDCl_3$): δ 0.833 (s, 3 H, C18-$CH_3$), 4.352 and 4.655 (AB, 2 H, $J_{AB}$ = 16.8 Hz, C21-$CH_2$) and 5.687 (s, 1 H, C4-CH=) MS (EI) m/z (relative intensity): 350 ($M^+$, 33.1), 348 (100.0), 253 (63.7), 213 (71.5) and 91 (62.6).

*Step 2. 17α-Hydroxy- 21-acetoxy-19-norpregna-4,9-diene-3,20-dione (116):*

The 21-chloro compound (115, 37.90 g, 108.64 mmol), KOAc (111.83 g, 1139.63 mmol) and acetonitrile (927 mL) were mechanically stirred. The suspension was brought to reflux under nitrogen. After 2.5 hr, TLC on silica (5% acetone in methylene chloride) indicated the reaction had gone to completion. The reaction mixture was allowed to cool to room temperature, and precipitated KCl was removed by filtration through a sintered glass funnel. Acetonitrile was evaporated in *vacuo,* and the resulting residue was taken up in $CH_2Cl_2$ and $H_2O$. The mixture was transferred to a separatory funnel, extracted with $CH_2Cl_2$ (3x), and washed with $H_2O$ (2x) and brine (1x). The organic fractions were combined, filtered through $Na_2SO_4$ and evaporated *in vacuo* to give 36.26 g of 116 in 89.61% crude yield. The solid material was taken up in hot acetone (150 mL) and $CH_2Cl_2$ (150 mL). The solution was scratched, seeded and stored in the freezer for 4 hr. The crystals were then filtered through a Buchner funnel and dried *in vacuo* to recover 10.71 g of the 17α-ol-21-acetate (116) in 52.14% yield. The mother liquor was evaporated *in vacuo* and purified by flash column chromatography eluted with 10% acetone in $CH_2Cl_2$. Fractions containing the 17α-ol-21-acetate (116) were combined and evaporated *in vacuo* to recover 2.58 g of a golden yellow solid in 12.61%. The total yield of the purified 17α-ol-21-acetate (116) was 13.29 g of a golden yellow solid in 64.7% yield; m.p. = 213 - 218°C. FTIR (KBr, diffuse reflectance): $v_{max}$ 3475, 2947, 2951, 1744, 1720, 1646, 1606, 1578, 1367 and 1235 $cm^{-1}$. NMR (300 MHz, $CDCl_3$): δ 0.841 (s, 3 H, C18-$CH_3$), 2.182 (s, 3 H, C21-OAc), 4.868 and 5.081 (AB, 2 H, $J_{AB}$ = 17.4 Hz, C21-$CH_2$) and 5.683 (s, 1H, C4-CH=) MS (EI) m/z (relative intensity): 372 ($M^{4+}$, 78.3), 354 (9.7), 312 (75.6), 253 (100.0) and 91 (69.3).

***Step 3. 17α,21-Dihydroxy-19-norpregna-4,9-diene-3,20-dione (117):***

The 17α-ol-21-acetate (116) (35.15 g, 94.37 mmol) was suspended in freshly opened MeOH (2870 mL) and deoxygenated by bubbling nitrogen through the mixture for 45 min. $KHCO_3$ (deoxygenated, 0.5 M, 283 mL, 141.74 mmol) was added, and the suspension was mechanically stirred and brought to reflux under nitrogen. After 10 minutes at reflux, TLC on silica (5% isopropanol in $CH_2Cl_2$) showed the reaction to be complete. The reaction mixture was cooled to room temperature, neutralized by the addition of HOAc (8.15 mL), and MeOH was evaporated *in vacuo*. The reaction mixture was extracted with $CH_2Cl_2$ (3x), and washed with $H_2O$ (2x) and brine (1x). The combined organic fractions were filtered through $Na_2SO_4$ and evaporated *in vacuo* to recover 29.83 g of the solid in 95.7% yield. The solid was taken up in acetone with a small amount of $CH_2Cl_2$. The solution was scratched, seeded and stored in the freezer for 1 hr. The resulting crystals were collected on a Buchner funnel, rinsed with acetone and dried *in vacuo* to recover the first crop. The mother liquor was concentrated and stored in the freezer overnight to afford a second crop of crystals. The combined solid recovered was 16.15 g in 51.8% crude yield. The mother liquors were evaporated *in vacuo* and purified by flash column chromatography eluted with 5% isopropanol in $CH_2Cl_2$. Fractions containing the diol (117) were combined and evaporated *in vacuo* to recover 4.86 g. The total yield of 117 was 19.75 g of a light yellow solid in 76.7%; m.p. = 197 - 204°C. FTIR (KBr, diffuse reflectance): $v_{max}$ 3917, 2954, 2869, 1715, 1635, and 1590 $cm^{-1}$. NMR (300 MHZ, $CDCl_3$): δ 0.827 (s, 3 H, C18-$CH_3$), 4.323 and 4.690 (AB, 2 H, $J_{AB}$ = 19.81 Hz, C21-$CH_2$) and 5.686 (s, 1 H, C4-CH=). MS (EI) m/z (relative intensity): 330 ($M^+$, 100.0), 312 (10.1), 253 (61.7), 213 (64.5), 174 (26.1) and 91. (38.5).

***Step 4. 3,20-bis-Ethylenedioxy-17α,21-Dihydroxy-19-norpregna-5(10),9(11)-diene (118):***

The diol (117, 9.88 g, 29.89 mmol) and freshlyopened ethylene glycol (750 mL) were magnetically stirred. *p*-Toluenesulfonic acid monohydrate (0.49 g, 2.60 mmol) was added to the suspension as a solid. The ethylene glycol was distilled *in vacuo* at 81°C under 2 mm Hg. After distilling for 3 hr, the mixture was cooled to room temperature and poured into saturated $NaHCO_3$ (250 mL) and $H_2O$ (250 mL). The mixture was extracted with CH $_2Cl_2$ (3x), washed with $H_2O$ (2x) and brine (1x). The organic fractions were combined, filtered through sodium sulfate and evaporated *in vacuo* to recover a solid. Analysis by TLC on silica (5% isopropanol in $CH_2Cl_2$) showed all of the starting material to be converted to an 85:15 mixture of 3,20-diketal to 3-ketal with a small amount of by-product. The resulting solid was triturated with ether, collected on a Buchner funnel, washed with additional ether and dried *in vacuo* to recover 6.46 g of 118 in 51.64% yield. The mother liquor was evaporated *in vacuo* and purified through flash chromatography eluting with 4% isopropanol in $CH_2Cl_2$. This recovered 0.6 g of the light beige, solid diketal in 4.8% yield. The total yield of the solid diketal (118) was 7.06 g of a light beige solid in 56.44% yield; m.p. =173 -176°C. FTIR (KBr, diffuse reflectance): $v_{max}$ 3452, 2892,1652, 1436, 1370, 1223 and 1055 $cm^{-1}$. NMR (300 MHz, $CDCl_3$): δ 0.795 (s, 3 H, C18-$CH_3$), 3.686 and 3.894 (AB, 2 H, $J_{AB}$ =12.61 Hz, C21-$CH_2$), 3.987 (s, 4 H, C3-$OCH_2CH_2O$-), 4.130 (m, 4 H, C20-$OCH_2CH_2O$-) and 5.555 (br s, 1 H, C11-CH=). MS(EI) m/z (relative intensity): 418 ($M^+$, 5.6), 400 (0.7), 387 (3.9), 314 (3.5), 211 (4.6) and 103 (100.0).

*Step 5.* 3,20-bis *Ethylenedioxy-17α-hydroxy-21-methoxy-19-norpregna-5(10),9(11)-diene (119):*

To a solution of the diketal (118, 0.5 g, 1.19 mmol) in $CH_2Cl_2$ (50 mL) was added 1,8-bis-(dimethylamino)naphthalene ("Proton Sponge", 1.28 g, 5.97 mmol) followed by trimethyloxonium tetrafluoroborate (0.88 g, 5.97 mmol). The mixture was mechanically stirred in an ice bath under nitrogen. The ice bath was allowed to melt to bring the reaction to room temperature. The reaction mixture was stirred for 3 hr, at which time TLC (5% isopropanol in $CH_2Cl_2$) indicated the reaction had gone to completion. The mixture was poured into a separatory funnel and washed with $H_2O$ (2x). The $CH_2Cl_2$ extracts (3x) were combined, filtered through $Na_2SO_4$ and evaporated *in vacuo.* The resulting residue was taken up in EtOAc, washed with ice-cold 1N HCl (2x), $H_2O$ (1x), saturated $NaHCO_3$ (1x), $H_2O$ (1x), and brine (1x). Combined EtOAc fractions (3x) were filtered through $Na_2SO_4$ and evaporated *in vacuo* to give 0.5 g of 119 as a yellow foam in 97.14% yield. The material was of adequate purity to carry onto the subsequent epoxidation. The reaction was repeated to produce a total of 13.57 g of the 21-methoxy compound (119). NMR (300 MHz, $CDCl_3$): δ 0.798 (s, 3 H, C18-$CH_3$), 3.415 (s, 3 H, C21-$OCH_3$), 3.546 and 3.715 (AB, 2 H, $J_{AB}$ = 10.51 Hz, C21-$CH_2$), 3.985 (s, 4 H, C3-$OCH_2CH_2O$-), 4.05 (m, 4 H, C20-$OCH_2CH_2O$-) and 5.54 (br s, 1 H, C11-CH=). Decomposition of analytical sample precluded further analysis.

***Step 6. 3,20-bis-Ethylenedioxy-5α,10α-epoxy-17α-hydroxy-21-methoxy-19-norpregn-9(11)-ene (120):***

Hexafluoroacetone trihydrate (6.49 mL, 46.64 mmol) and $CH_2Cl_2$ (100 mL) were mechanically stirred vigorously at 4°C. Solid $Na_2HPO_4$ (3.67 g, 25.91 mmol) and 30% $H_2O_2$ (7.01 mL, 68.39 mmol) were added and stirred for 15 minutes at 4°C. A cold solution of the 21-methoxy compound (119, 13.45 g, 31.09 mmol) in $CH_2Cl_2$ (100 mL) was added to the mixture via an additional funnel over a period of 1 hr. The reaction mixture was allowed to stir overnight at 4°C. Examination by TLC (25% EtOAc in $CH_2Cl_2$) showed all of the starting material had been converted to a mixture of the α and β epoxides in about a 2:1 ratio. The mixture was transferred to a separatory funnel and washed with 10% $Na_2SO_3$ (1x), saturated $NaHCO_3$ (1x) and $H_2O$ (1x), Combined $CH_2Cl_2$ extracts (3x) were filtered through $Na_2SO_4$ and evaporated *in vacuo* to recover 14.06 g of the epoxide (120) as a white foam in quantitative yield. The 2:1 mixture of α- and β-epoxides was used directly for the subsequent Grignard reaction. NMR (300 MHz, $CDCl_3$): δ 0.700 (s, 3 H, C18-$CH_3$), 3.407 (s, 3 H, C21-$OCH_3$), 3.539 and 3.692 (AB, 2 H, $J_{AB}$ = 10.51 Hz, C21-$CH_2$), 4.051 (m, 8 H, C3- and C20-$OCH_2CH_2O$-), 5.819 (br s, 0.3 H, C11-CH= of β-epoxide), and 5.997 (br s, 0.6 H, C11-CH= of α-epoxide). Decomposition of analytical sample precluded further analysis.

*Step 7. 3,20-bis-Ethylenedioxy-5α,17α-dihydroxy-11β-[4-(2-methyl-1,3-dioxolan-2-yl)phenyl]-21-methoxy-19-norpregn-9-ene (121b):*

A 3-neck 1 L flask was euipped with a mechanical stirrer, an addition funnel, and a reflux condenser and flame-dried under a stream of nitrogen, Magnesium (3.90 g, 146 mmol) was added, followed by one iodine crystal, 150 mL of dry THF, and 1-2 drops of 1,2-dibromoethane. The mixture was stirred under nitrogen and heated in a warm water bath, but no reaction occurred, 4-Bromoacetophenone ethylene ketal (*see,* EXAMPLE 1, Step 3) (35.5 g, 146 mmol) was added as a solution in THF (100 mL) via the addition funnel and then rinsed in with additional THF (40 mL). Upon completion of addition, the mixture was heated to reflux to initiate formation of the Grignard reagent. Heating was discontinued and the mixture allowed to stir 1.5 hr as the water bath gradually cooled to room temperature. Copper (I) chloride (1.59 g, 16.06 mmol) was added as a solid and stirring continued for another ½ hr. The mixture of epoxides (120, 13.11 g, 29.2 mmol, ~66% α-epoxide) was added as a solution in THF (50 mL) via the addition funnel and rinsed in with additional THF (20 mL). After stirring 1.5 hr at room temperature, TLC (20% acetone in $CH_2Cl_2$; quenched with saturated $NH_4Cl$ and extracted into EtOAc) indicated the reaction was >95% complete. The reaction was quenched by the addition of 200 mL of saturated $NH_4Cl$ and air was drawn through the mixture for ½ hr with vigorous stirring. Ether was added, the mixture was transferred to a separatory funnel, and the layers allowed to separate. The organic layer was washed with 10% $NH_4Cl$, $H_2O$ and brine. Combined ether extracts (3x) were filtered through $Na_2SO_4$ and evaporated in *vacuo* to give 35.23 g of the crude product (121b). Purification by flash column chromatography (20% acetone in $CH_2Cl_2$) afforded 7.24 g of a pale foam. Trituration of this foam with ether and pentane produced 5.93 g of the product (121b) as a beige powder in 50.2% yield (based on 66% of the mixture as α-epoxide). NMR ($CDCl_3$): δ 0.4 (s, 3 H, C18-$CH_3$), 1.63 (s, 3 H, $CH_3$ of C11β-4-$C_6H_4C(O)CH_3$), 3.45 (s, 3 H, C21-$OCH_3$), 3.57 - 4.40 (m, 15 H, C3-$OCH_2CH_2O$-, C11β-$OCH_2CH_2O$- and C20- $OCH_2CH_2O$-, C11α-CH and C21-$CH_2$-), 7.2 (d, 2 H, J = 9 Hz, 2', 6' aromatic-CH's) and 7.83 (d, 2 H, J = 9 Hz, 3', 5' aromatic-CH's). MS (EI) m/z (relative intensity): 612 ($M^+$, 0.1), 594 (3.3), 549 (15.0), 459 (2.7), 117 (100.0) and 87 (74.7). Decomposition of the analytical sample precluded further analysis.

*Step 8. -17α-Hydroxy-11β-(4-acetylphenyl)-21-methoxy-19-norpregna-4,9-diene-3,20-dione (122b):*

The Grignard adduot (121b, 5.81 g, 9.48 mmol) was dissolved in THF (60 mL) and stirred magnetically, under nitrogen, at room temperature. Trifluoroacetic acid (180 mL) was added followed by $H_2O$ (60 mL). After 1.5 hr, examination by TLC (20% acetone in $CH_2Cl_2$; neutralized with $NH_4OH$ before developing) indicated all of the starting material had been converted to a slightly less polar product. The reaction mixture was neutralized by the careful addition of $NH_4OH$

(165 mL) *via* an addition funnel. Enough additional $NH_4OH$ was added to bring the pH to 7.0 by pH paper. $H_2O$ was added, the mixture was transferred to a separatory funnel, and extracted with BtOAo. The organic fraction was washed again with $H_2O$ and brine. Combined EtOAc fractions (3x) were filtered through $Na_2SO_4$ and evaporated *in vacuo* to give *6.60* g of a foam. Purification of the crude product by flash column chromatography (20% acetone in $CH_2Cl_2$) afforded a yellow solid (122b). Crystallization from a minimum amount of hot EtOAc gave large, bright yellow crystals. The crystals wore collected on a Buchner funnel and dried overnight under high vacuum at 70°C to recover 2.84 g of 122b. A TLC of the mother liquors indicated they were pure enough to carry on to the subsequent reaction. The mother liquors were evaporated *in vacuo* and dried under high vacuum over the weekend to recover 0.46 g. The total yield of the 17α-OH (122b) was 3.3 g as bright yellow crystals in 75.25% yield. A small amount of the crystalline product was dried *in vacuo* at 110°C over the weekend for purposes of characterization. The crystals were fused and pulverized with a spatula; m.p. = 105 - 109°C (softens). Analysis by HPLC on a Phenomenex Prodigy 5 ODS-2 column (150 x 4.6 mm) oluted with 50% $CH_3CN$ in $H_2O$ at a flow rate of 1 mL per min and λ = 302 nm indicated a purity of >99% with a retention time ($t_R$) of 5.02 min. FTIR (KBr, diffuse reflectance): $v_{max}$ 3444, 2944, 1722, 1662, 1602, 1407 1359 and 1.271 $cm^{-1}$. NMR ($CDCl_3$): δ 0.33 (s, 3 H, C18-$CH_3$), 2.57 (s, 3 H, C11β-4-$C_6H_4$-C(O)$CH_3$), 3. 47 (s, 3 H, C21-$OCH_3$), 4.23 - 4.47 (AB, 2 H, $J_{AB}$ = 18 Hz, C21-$CH_2$-), 4.52 (br d, 1 H, C11α-CH), 5.48 (s, 1 H, C4-CH=), 7.3 (d, 2 H, J = 9 Hz, 2', 6' aromatic-CH's) and 7.92 (d, 2 H, J = 9 Hz, 3', 5' aromatic-CH's). MS (EI) m/z (relative intensity): 462 ($M^+$, 100.0), 430 (11.2), 389 (27.0), 346 (97.9) and 91 (22.3). Anal. Calcd. for $C_{29}H_{34}O_5 \cdot 9/20C_4H_8O_2$: C, 73.66; H, 7.55. Found: C, 73.66; H, 7.29.

*Step 9. |Preparation of the target compound 123b:*

A mixture of trifluoroacetic anhydride (32.78 g, 156 mmol) and acetic acid (9.38 g, 156 mmol) in $CH_2Cl_2$ (100 mL) was allowed to stir ½ hr at room temperature under nitrogen. The mixture was cooled to 0°C in an ice $H_2O$ bath and *p*-toluenosulfonic acid monohydrate (1.30 g, 6.86 mmol) was added as a solid. The 17α-OH (122b, 2.89 g, 6.24 mmol) was added as a solution in 25 mL of $CH_2Cl_2$ and rinsed in with additional $CH_2Cl_2$ (25 mL). After 45 min, TLC (10% acetone in $CH_2Cl_2$) indicated the reaction had gone to completion. The reaction was neutralized by the careful addition of $NH_4OH$ (31.6 mL, 416 mmol). Additional $NH_4OH$ was added to bring the pH to 7 by pH paper. Water was added and the mixture transferred to a separatory tunnel. The organic fractions were washed with $H_2O$ and brine. Combined $CH_2Cl_2$ extracts (3x) were filtered through $Na_2SO_4$ and evaporated *in vacuo* to recover 3.13 g of crude material, Purification by flash chromatography (10% acetone in $CH_2Cl_2$) provided 1.56 g of a crystallizing oil. Additional fractions containing a small amount of a less polar impurity were also combined and evaporated to give 1.04 g of an oil. Pure fractions were crystallized from a minimum amount of boiling EtOAc, triturated with pentane and dried 3 nights in a drying pistol at 110°C to give 0.99 g of 123b as pale yellow crystals. The crystals fused at this temperature, but were readily pulverized for analysis. Mother liquors were combined with the impure fractions and crystallized from EtOAc to give an additional 0.9 g. Total yield of 123b was 1.89 g as a pale yellow solid in 60.1% yield; mp. = 113°C (softens).

Analysis by HPLC on a Phenomenex Prodigy 5 ODS-2 column (150 x 4.6 mm) eluted with 50% $CH_3CN$ in $H_2O$ at a flow rate of 1 mL per min and λ = 302 nm indicated a purity of 99.7% with a retention time ($t_R$) of 7.69 min. FTIR (KBr, diffuse reflectance): $v_{max}$ 2942, 1730, 1680,1602, 1432,1408,1368 and 1266 $cm^{-1}$, NMR ($CDCl_3$): δ 0.33 (s, 3 H, C18-$CH_3$), 2.10 (s, 3 H, C17α-OAc), 2.57 (s, 3 H, C11β-C(O)$CH_3$), 3. 42 (s, 3 H, C21-$OCH_3$), 4.07 & 437 (AB, 2 H, $J_{AB}$ = 18·Hz, C21-$CH_2$-), 4.50 (brd, 1 H, C11α-CH), 5.83 (*s*, 1 H, C4-CH=), 7.28 (d, 2H, J = 9 Hz, 2', 6' aromatic-CH's) and 7.92 (d, 2 H, J =9 Hz, 3',5' axomatic-CH's). MS (EI) m/z (relative intensity): 504 ($M^+$, 3,3), 447 (17.9), 389 (28.4), 371 (100.0) and 91 (13.8). Anal. Calcd. for $C_{31}H_{36}O_6 \cdot 1/6CH_2Cl_2 \cdot 1/2H_2O$: C, 70.92; H, 7.13. Found: C, 71.06; H, 6.91.

<u>**Biological Properties of the Compounds of the invention**</u>

**MATERIALS AND METHODS**

***Statistical Analysis***

Statistical analysis was performed using standard methods and a PROPHET data management system operating on SUN Microsystems OS 4.4.1, (Bliss, Cl., The Statistics of Bioassay, New York, Academic Press (1952); Hollister, C., Nucleic Acids Research, 16:1873-1875 (1988)). Raw data, statistical and regression analysis are available.

***AntiMcGinty Test (McGinty, et al., Endocrinology, 24:829-832 (1939))***

Immature female rabbits of the New Zealand White breed (approx. 1 kg body weight) were maintained under standard laboratory conditions and received a subcutaneous injection of 5 μg estradiol in 10% ethanol/sesame oil daily for 6 consecutive days. Twenty-four hours after the last injection of estradiol (day 7) animals underwent sterile abdominal surgery to ligate a 3-4 cm segment of both uterine horns. The experimental compound in appropriate solvent (usually

10% ethanol/sesame oil) was injected intraluminally into the ligated segment of one uterine horn and the vehicle alone into the ligated segment of the contralateral horn. Injection volume was limited to 0.1 ml, and care was taken to prevent leakage. A stimulating dose of progesterone (0.8 mg/day) was administered subcutaneously to each rabbit daily for the next three days (days 7, 8 and 9) for the purpose of inducing endometrial proliferation. All animals were sacrificed on day 10 when a segment central to the ligatures was removed and fixed in 10% neutral buffered formalin and submitted for histological processing. Five micron sections stained with hematoxylin and eosin (H&E) were evaluated microscopically for the degree of endometrial glandular proliferation according to the method of McPhail (McPhail, *J. Physiol.*, *8*3:145 (1934). The percent inhibition of endometrial proliferation for each rabbit was calculated and the mean of the group of five animals recorded.

***AntiClauberg Test (Clauberg, C., Zentr. Gynakol., 54:2757-2770 (1930))***

Immature female rabbits of the New Zealand White breed (approx. 1 kg body weight) were maintained under standard laboratory conditions and received a subcutaneous injection of 5 μg estradiol in 10% ethanol/sesame oil daily for 6 consecutive days. Twenty-four hours after the last dose of estradiol (day 7) animals received progesterone by subcutaneous injection (0.8 mg/day) and the experimental compound in appropriate vehicle (usually 10% ethanol/sesame oil) orally or subcutaneously for five consecutive days. One group of rabbits received progesterone only. Twenty-four hours after the last dose all animals were sacrificed for removal of the uterus which was cleaned of all fat and connective tissue, weighed to the nearest 0.2 mg and placed in 10% neutral buffered formalin for subsequent histological processing. Five micron sections stained with hematoxylin and eosin (H&E) were evaluated microscopically for the degree of endometrial glandular proliferation according to the method of McPhail (McPhail, *supra*). The percent inhibition of endometrial proliferation at each dose level of the experimental compound was derived by comparison with the progesterone-stimulated animals alone.

***Postcoital Test***

Adult female rats of the Sprague-Dawley strain were maintained under standard laboratory conditions, 14 hours of light and 10 hours of darkness each day and cohabited with proven fertile males when in proestrus. Sperm-positive animals were randomly assigned to control and experimental groups. The day vaginal sperm were found in vaginal washings constituted day 0 of gestation. Rats received experimental compounds or vehicle (control) daily by the oral route on days 0-3 or 4-6. and were sacrificed between days 10 and 17 to record the number and condition of conceptuses.

***Antiovulatory Test***

Immature female rats of the Sprague-Dawley strain weighing 200 to 250 g were maintained under standard laboratory conditions, 14 hours of light and 10 hours of darkness each day. Vaginal washings were obtained daily and evaluated microscopically to establish the estrous cycle of each animal. Animals exhibiting two consecutive four-day cycles were used in the test Each dose group consisted of eight rats and one group served as the vehicle control. Animals were dosed at noon on the day of proestrus and sacrificed 24 hours later when ova can usually be visualized in the distended ampulla of the oviduct using a dissecting microscope. The oviducts were excised, an incision made in the distended ampulla and the ova teased out in a drop of water on a microscope slide so that the number shed could be counted. Historically, control animals shed between 12 and 14 ova during each estrous cycle. Agents which inhibit ovulation usually exhibit an "all or none" effect; it is rare that ovulation is "partially" inbibited. Treatment groups were compared with the control group using a 95% contingency table or the $ED_{100}$ was established with additional dose levels.

***Relative Binding Affinities for the Progesterone and Glucocorticoid Receptors***

Uteri and thymus glands were obtained from estradiol-primed immature female rabbits of the New Zealand White strain for preparation of cytosols for the progesterone and glucocorticoid receptor assays, respectively. Tissues were excised and immediately placed in ice cold TEGDM buffer (10 mM Tris, pH 7.4; 1.5 mM EDTA; 10% glycerol vol/vol/; 1 mM dithiothreitol [DTT]; and 20 mM sodium molybdate). The tissues were dissected free of connective tissue and fat, weighed and minced finely. Minced tissues were homogenized in 3 volumes TEGDM/gm with four 10 second bursts of a VirTis Cyclone set at half maximum speed with a 30 second cooling period (in ice) between bursts. Homogenates were centrifuged at 109,663 g at 4°C for 1 hour to yield the soluble cytosol fraction. Aliquots of cytosol were snap frozen and stored at -75°C.

All binding assays were carried out at 2-6°C for 16-18 hours. The following radioactive ligands were used: [1,2-$^3$H(N)]-progesterone (50.0 Ci/mmole) for the progesterone receptor (PR). [6,7-$^3$H(N)-dexamethasone (39.2 Ci/mmole) for the glucocorticoid receptor (GR) and [2,4,6,7-$^3$H(N)]-estradiol for the estrogen receptor. For the progesterone receptor

RBA assays 0.02 ml uterine cytosol or TEDGM buffer, 0.05 ml of various concentrations of test compounds or progesterone, 0.13 ml TEGDM buffer and 0.05 ml [$^3$H]-progesterone were added to duplicate tubes. For the glucocorticoid receptor RBA assays 0.1 ml thymus cytosol or TEDGM buffer, 0.05 ml of various concentrations of test compounds or dexamethasone, 0.05 ml TBGDM buffer and 0.05 ml [$^3$H]-dexamethasone were added to duplicate tubes. For the estrogen receptor RBA assays 0.05 ml uterine cytosol, 0.1 ml TEGDM buffer, 0.05 ml of various concentrations of test compounds or estradiol and 0.05 ml [$^3$H-estradiol were added to duplicate tubes. The concentrations of the test compounds, progesterone, dexamethasone and estradiol ranged from 0.05 to 100 nM and the concentrations of the competitors ranged from 0.5 to 500 nM. Total binding was measured at radioligand concentrations of 3.5 nM and nonspecific binding was measured in the presence of a 200-fold excess of unlabeled progesterone (PR), dexamethasone (GR) or diethylstilbestrol (BR), respectively.

In all incubations bound and free ligand were separated using extra-coated charcoal (DCC). A 0.1 ml aliquot of DCC (0.5% charcoal/0.05% DextranT-70) was added to each tube. The tubes were vortexed and incubated on ice for 10 minutes. Five-tenths ml TEG buffer (without DTT or molybdate) was then added to all tubes to improve supernatant recovery following centrifugation. The charcoal was pelleted by centrifugation at 2,100 g for 15 minutes at 4°C. The supernatants containing the [$^3$H]-steroid receptor complexes were decanted into vials containing 4 ml Optifluor (Packard Instrument Co.), vortexed, equilibrated in a liquid scintillation counter for 30 minutes and then counted for 2 minutes. This provided the quantity of receptor bound [$^3$H]-steroid at each competitor concentration.

The standard curves and the $EC_{50}$ (Effective Concentration) for each standard curve and curve for each test compound was determined by entering the counting data (receptor bound [$^3$H]-progesterone, [$^3$H]-dexamethasone or [$^3$H]-estradiol) into a four parameter sigmoidal computer program (RiaSmart® Immunoassay Data Reduction Program, Packard Instrument Co., Meriden, Connecticut. The RBA for each test compound was calculated using the following equation:

$$RBA = \frac{EC_{50}\ \text{Standard}}{EC_{50}\ \text{Test Compound}} X\ 100$$

where $EC_{50}$ Standard = molar concentration of unlabeled progesterone, dexamethasone or estradiol required to decrease bound [$^3$H]-progesterone (PR), [$^3$H]-dexamethasone (GR) or [$^3$H]-estradiol to 50% of the respective buffer control (100% bound radioligand) and $EC_{50}$ Test Compound = molar concentration of test compound required to decrease bound [$^3$H]-progesterone (PR), [$^3$H]-dexamethasone (GR) or [$^3$H]-estradiol to 50% of the respective buffer control (100% bound radioligand).

RESULTS

EXAMPLE 1

Since mifepristone (CDB-2477) is frequently used as a reference standard, Table 2, *infra*, contains data comparing the antiprogestational activity and relative binding affinity for the progesterone and glucocorticoid receptors of CDB-2914 with this standard. Recent studies have shown a good correlation between relative binding affinity for the glucocorticoid receptor and a biological test based upon the antagonism of dexamethasone-induced thymus involution in adrenalectomized male rats.

**Table 2**

| CDB NO. | COMPOUND NO. | BINDING AFFINITY[1] | | BIOLOGICAL ACTIVITY | | |
|---|---|---|---|---|---|---|
| | | Progester | Glucocortic | antiClauberg[2] | Postcoital[3] | Antiovulator[4] |
| 2914 | 69B | 122 (234) | 114 | 100 | 2 | 1 |
| 3875 | 69A | 164 | 30 | 97 | | |
| 3247 | 69C | 91 | 49 | ~10 | 2* | |
| 3248 | 69D | 40 | 89 | weak (subcu) | inactive @2* | |
| 4243 | 91 | 171 | 59 | inactive | | |
| 4418 | 70 | 79 | /2 | ~25 | | |
| 4363 | 71 | 123 (203) | 20 | 253 | 0.5 | >16 |

(continued)

| CDB NO. | COMPOUND NO. | BINDING AFFINITY[1] | | BIOLOGICAL ACTIVITY | | |
|---|---|---|---|---|---|---|
| | | Progester | Glucocortic | antiClauberg[2] | Postcoital[3] | Antiovulator[4] |
| 4399 | 72 | 109 | 110 | 35 | | |
| 4176 | 74 | 131 | 32 | <10 | | |
| 4324 | 97a | 120 | 52 | 110 | | |
| 4398 | 97b | 47 | 38 | 99 | | |
| 4455 | 106a | | | | | |
| 4241 | 106b | 136 (172) | 14 | 34 | | |
| 4400 | 113A | 117 (237) | 62 | 229 | | |
| 4454 | 113B | 59 | 34 | | | |
| 4417 | 113c | 63 | 45 | 70 | | |
| 4239 | 123a | 174 (140) | 11 | 45-83 | | |
| 4416 | 123b | 64 | 45 | 77 | | |
| 4393 | 139 | 30 | 79 | inactive | | |
| 4247 | 126a | 95 | 43 | 170 | | |
| 4362 | 126b | 76 | 15 | 125 | | |
| 4374 | 126c | 68 | 67 | 224 | | |
| 4361 | 129 | 155 | 20 | 303 | | |
| 4306 | 133 | 82 | 13 | 95 | | |
| 4352 | 138 | 63 | 14 | 57 | | |

[1]Progesterone receptor (estrogen-primed rabbit uterus); progesterone =100% Figure in 0 is relative binding affinity of the human isoform A progesterone receptor Glucocorticoid receptor (estrogen-primed rabbit thymus) dexamethasone = 100%.
[2]antiClaubarg - oral except where indicated; CDB-2914 = 100 (assigned).
[3]Postcoital - oral, rat $MED_{100}$ (mg/day) days 0-3 or *days 4-6 subcu; day sperm in vaginal washings = day 0.
[4]Antiovulatory - oral, rat $MED_{100}$(mg) single dose at noon on day of proestrus.

**EXAMPLE 2**

***AntiClauberg***

Data from antiClauberg tests following oral administration are shown in Tables 1 and 2. Compounds 15, 38, 40, 41, 46, 71, 97a, 113a, 126a, 126b, 126c and 129 exhibited greater activity than the standard, 69B. Previous studies have shown that 69B is significantly more potent than mifepristone (3.27 X; 95% C.I. = 1.41-7.58) in this test. Compounds 15, 38, 71 and 129 represent four of the most potent antiprogestational compounds known, and their low binding affinity for the glucocorticoid receptor would predict minimal antiglucocorticoid activity.

*Posicoital*

Compound 71 exhibited about four times the postcoital contraceptive activity of the standard, compound 69B, following oral administration on days 0-3 of gestation.

*Antiovulatory*

Compound 71 was not fully active at a dose level 16 times the $MBD_{100}$ for the standard, compound 69B, and compound 113a exhibited only about 6% of the antiovulatory activity of the standard.

*Relative Binding Affinity for the Progesterone and Glucocorticoid Receptors*

Relative binding affinities for the progesterone receptor (estrogen-primed rabbit uterine cytosol) and glucocorticoid receptor (estrogen-primed rabbit thymic cytosol) are shown in Table 1. Several compounds were also tested for binding affinity for the human isoform A progesterone receptor. Compounds 12, 13, 14A, 14B, 15, 28, 38, 69A, 91, 71, 71, 73, 97a, 106b, 113a, 113d, 122b and 129 showed binding affinities greater than that observed for the standard, compound 69B. On the other hand, most of the compounds tested exhibited reduced binding affinity for both the progesterone and the glucocorticoid receptor.

## DISCUSSION

Many members of a series of derivatives of 19-norprogesterone possess potent antiprogestational activity following oral administration in experimental animals. They exhibit high binding affinity for the progesterone receptor (rabbit uterine) and only modest relative binding affinity for the glucocorticoid receptor (rabbit thymus). This is reflected in standard antiprogestational assays showing strong inhibition of progesterone-induced alterations of rabbit uterine endometrium. It is anticipated that the reduced binding affinity for the glucocorticoid receptor will reflect diminished biological antiglucocorticoid activity.

## Claims

1. A compound having the general formula:

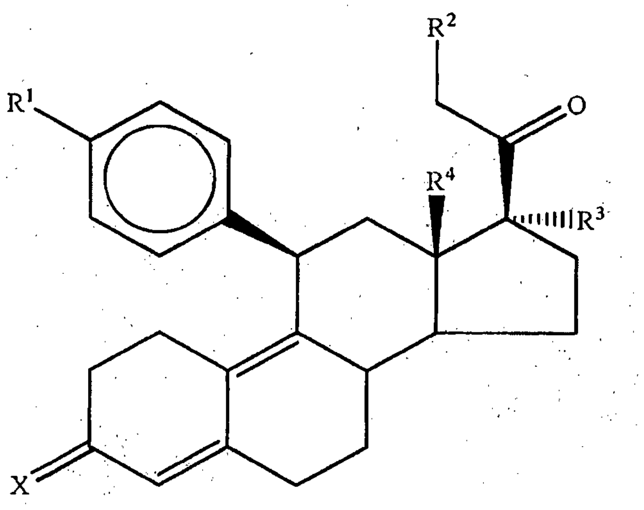

wherein

$R^1$ is $-C(O)CH_3$;
$R^2$ is acetoxy;
$R^3$ is acetoxy;
$R^4$ is ethyl; and
X is =O;
$R^1$ is $-C(O)CH_3$;
$R^2$ is -SAc;
$R^3$ is acetoxy;
$R^4$ is methyl; and
X is =O;
$R^1$ is $-C(O)CH_3$;
$R^2$ is methoxy;
$R^3$ is methoxy;
$R^4$ is methyl; and
X is =O;

$R^1$ is -C(O)$CH_3$;
$R^2$ is methoxy;
$R^3$ is acetoxy;
$R^4$ is methyl;
X is =O;
$R^1$ is -C(O)$CH_3$;
$R^2$ is H;
$R^3$ is OH;
$R^4$ is methyl; and
X is =O;
$R^1$ is -C(O)$CH_3$;
$R^2$ is Br;
$R^3$ is OH;
$R^4$ is methyl; and
X is =O;
$R^1$ is -C(O)$CH_3$;
$R^2$ is acetoxy;
$R^3$ is OH;
$R^4$ is methyl; and
X is =O;
$R^1$ is -C(O)$CH_3$;
$R^2$ is -SAc;
$R^3$ is OH;
$R^4$ is methyl; and
X is =O; or
$R^1$ is -C(O)$CH_3$;
$R^2$ is methoxy;
$R^3$ is OH;
$R^4$ is methyl; and
X is =O.

**2.** The compound of claim 1, wherein:

$R^1$ is -C(O)$CH_3$;
$R^2$ is acetoxy;
$R^3$ is acetoxy;
$R^4$ is methyl; and
X is =O.

**3.** The compound of claim 1, wherein:

$R^1$ is -C(O)$CH_3$;
$R^2$ is -SAc;
$R^3$ is acetoxy;
$R^4$ is methyl; and
X is =O.

**4.** The compound of claim 1, wherein:

$R^1$ is -C(O)$CH_3$;
$R^2$ is methoxy;
$R^3$ is methoxy;
$R^4$ is methyl; and
X is =O.

**5.** The compound of claim 1, wherein:

$R^1$ is -C(O)$CH_3$;

$R^2$ is methoxy;
$R^3$ is acetoxy;
$R^4$ is methyl; and
X is =O.

**6.** A pharmaceutical composition comprising an effective amount of a compound in accordance with any one of claims 1 to 5 and a pharmaceutically acceptable excipient.

**7.** A compound in accordance with any one of claims 1 to 5 or the pharmaceutical composition in accordance with claim 6, for use in producing an antiprogestational effect in a patient.

**8.** A compound in accordance with any one of claims 1 to 5 or the pharmaceutical composition in accordance with claim 6, for use in

(a) inducing menses;
(b) treating endometriosis;
(c) treating dysmenorrhea;
(d) treating an endocrine hormone-dependent tumor;
(e) treating meningiomas;
(f) treating uterine fibroids;
(g) inhibiting uterine endometrial proliferation; or
(h) inducing labor in a patient.

**9.** Use of a compound in accordance with any one of claims 1 to 5 or the pharmaceutical composition in accordance with claim 6 in the preparation of a contraceptive.

**10.** Use of a compound in accordance with any one of claims 1 to 5 or the pharmaceutical composition in accordance with claim 6 in the preparation of a postcoital contraceptive.

**Patentansprüche**

**1.** Eine Verbindung mit der allgemeinen Formel:

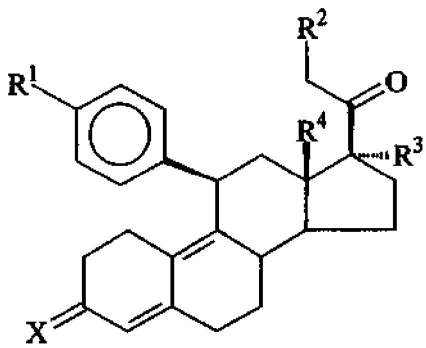

worin

$R^1$ ist $-C(O)CH_3$;
$R^2$ ist Acetoxy;
$R^3$ ist Acetoxy;
$R^4$ ist Methyl; und
X ist =0;
$R^1$ ist $-C(O)CH_3$;
$R^2$ ist -SAc;
$R^3$ ist Acetoxy;
$R^4$ ist Methyl; und

X ist =0;
$R^1$ ist -C(O)$CH_3$;
$R^2$ ist Methoxy;
$R^3$ ist Methoxy;
$R^4$ ist Methyl; und
X ist =0;
$R^1$ ist -C(O)$CH_3$;
$R^2$ ist Methoxy;
$R^3$ ist Acetoxy;
$R^4$ ist Methyl;
X ist =0;
$R^1$ ist -C(O)$CH_3$;
$R^2$ ist H;
$R^3$ ist OH;
$R^4$ ist Methyl; und
X ist =0;
$R^1$ ist -C(O)$CH_3$;
$R^2$ ist Br;
$R^3$ ist OH;
$R^4$ ist Methyl; und
X ist = O;
$R^1$ ist -C(O)$CH_3$;
$R^2$ ist Acetoxy;
$R^3$ ist OH;
$R^4$ ist Methyl; und
X ist =0;
$R^1$ ist -C(O)$CH_3$;
$R^2$ ist -SAc;
$R^3$ ist OH;
$R^4$ ist Methyl; und
X ist = O; oder
$R^1$ ist -C(O)$CH_3$;
$R^2$ ist Methoxy;
$R^3$ ist OH;
$R^4$ ist Methyl; und
X ist = O.

**2.** Die Verbindung nach Anspruch 1, worin:

$R^1$ ist -C(O)$CH_3$;
$R^2$ ist Acetoxy;
$R^3$ ist Acetoxy;
$R^4$ ist Methyl; und
X ist = O.

**3.** Die Verbindung nach Anspruch 1, worin:

$R^1$ ist -C(O)$CH_3$;
$R^2$ ist -SAc;
$R^3$ ist Acetoxy;
$R^4$ ist Methyl; und
X ist = O.

**4.** Die Verbindung nach Anspruch 1, worin:

$R^1$ ist -C(O)$CH_3$;
$R^2$ ist Methoxy;
$R^3$ ist Methoxy;

$R^4$ ist Methyl; und
X ist = O.

**5.** Die Verbindung nach Anspruch 1, worin:

$R^1$ ist $-C(O)CH_3$;
$R^2$ ist Methoxy;
$R^3$ ist Acetoxy;
$R^4$ ist Methyl; und
X ist = O.

**6.** Eine pharmazeutische Zusammensetzung, umfassend eine wirksame Menge einer Verbindung gemäß mindestens einem der Ansprüche 1 bis 5 und einem pharmazeutisch annehmbaren Hilfsstoff.

**7.** Eine Verbindung gemäß mindestens einem der Ansprüche 1 bis 5 oder die pharmazeutische Zusammensetzung nach Anspruch 6 zur Verwendung bei der Hervorrufung eines antiprogestationalen Effektes in einem Patienten.

**8.** Eine Verbindung nach mindestens einem der Ansprüche 1 bis 5 oder die pharmazeutische Zusammensetzung gemäß Anspruch 6 zur Verwendung für das:

(a) Induzieren von Menstruationen;
(b) Behandeln von Endometriose;
(c) Behandeln von Dysmenorrhö;
(d) Behandeln eines endokrinen Hormon-abhängigen Tumors;
(e) Behandeln von Meningiomata;
(f) Behandeln von Uterusfibroiden;
(g) Inhibieren der Uterus-endometrialen Proliferation; oder
(h) Induzieren von Wehen in einem Patienten.

**9.** Verwendung einer Verbindung nach mindestens einem der Ansprüche 1 bis 5 oder der pharmazeutischen Zusammensetzung nach Anspruch 6 zur Herstellung eines Kontrazeptivums.

**10.** Verwendung einer Verbindung nach mindestens einem der Ansprüche 1 bis 5 oder der pharmazeutischen Zusammensetzung nach Anspruch 6 zur Herstellung eines postkoitalen Kontrazeptivums.

## Revendications

**1.** Composé de formule générale

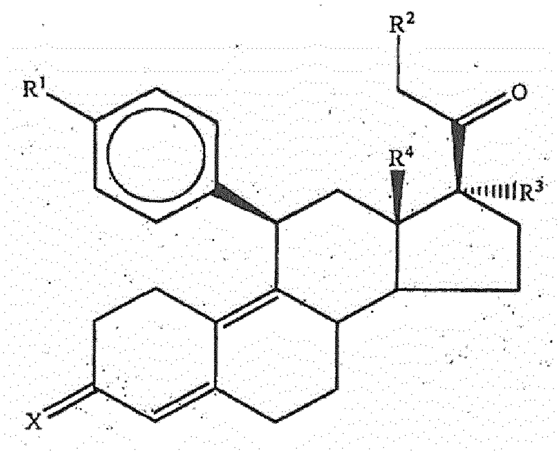

dans laquelle

- $R^1$ représente un groupe acétyle $-C(O)CH_3$,
- $R^2$ représente un groupe acétoxy,
- $R^3$ représente un groupe acétoxy,
- $R^4$ représente un groupe méthyle,
- et X représente un substituant oxo (=O) ;

ou bien

- $R^1$ représente un groupe acétyle $-C(O)CH_3$,
- $R^2$ représente un groupe acétyl-thio (-S-Ac),
- $R^3$ représente un groupe acétoxy,
- $R^4$ représente un groupe méthyle,
- et X représente un substituant oxo (=O) ;

ou bien

- $R^1$ représente un groupe acétyle $-C(O)CH_3$,
- $R^2$ représente un groupe méthoxy,
- $R^3$ représente un groupe méthoxy,
- $R^4$ représente un groupe méthyle,
- et X représente un substituant oxo (=O) ;

ou bien

- $R^1$ représente un groupe acétyle $-C(O)CH_3$,
- $R^2$ représente un groupe méthoxy,
- $R^3$ représente un groupe acétoxy,
- $R^4$ représente un groupe méthyle,
- et X représente un substituant oxo (=O) ;

ou bien

- $R^1$ représente un groupe acétyle $-C(O)CH_3$,
- $R^2$ représente un atome d'hydrogène,
- $R^3$ représente un groupe hydroxyle,
- $R^4$ représente un groupe méthyle,
- et X représente un substituant oxo (=O) ;

ou bien

- $R^1$ représente un groupe acétyle $-C(O)CH_3$,
- $R^2$ représente un atome de brome,
- $R^3$ représente un groupe hydroxyle,
- $R^4$ représente un groupe méthyle,
- et X représente un substituant oxo (=O) ;

ou bien

- $R^1$ représente un groupe acétyle $-C(O)CH_3$,
- $R^2$ représente un groupe acétoxy,
- $R^3$ représente un groupe hydroxyle,
- $R^4$ représente un groupe méthyle,
- et X représente un substituant oxo (=O) ;

ou bien

- $R^1$ représente un groupe acétyle $-C(O)CH_3$,
- $R^2$ représente un groupe acétyl-thio (-S-Ac),

- $R^3$ représente un groupe hydroxyle,
- $R^4$ représente un groupe méthyle,
- et X représente un substiuant oxo (=O) ;

ou bien

- $R^1$ représente un groupe acétyle $-C(O)CH_3$,
- $R^2$ représente un groupe méthoxy,
- $R^3$ représente un groupe hydroxyle,
- $R^4$ représente un groupe méthyle,
- et X représente un substiuant oxo (=O).

**2.** Composé conforme à la revendication 1, dans lequel

- $R^1$ représente un groupe acétyle $-C(O)CH_3$,
- $R^2$ représente un groupe acétoxy,
- $R^3$ représente un groupe acétoxy,
- $R^4$ représente un groupe méthyle,
- et X représente un substituant oxo (=O).

**3.** Composé conforme à la revendication 1, dans lequel

- $R^1$ représente un groupe acétyle $-C(O)CH_3$,
- $R^2$ représente un groupe acétyl-thio (-S-Ac),
- $R^3$ représente un groupe acétoxy,
- $R^4$ représente un groupe méthyle,
- et X représente un substituant oxo (=O).

**4.** Composé conforme à la revendication 1, dans lequel

- $R^1$ représente un groupe acétyle $-C(O)CH_3$,
- $R^2$ représente un groupe méthoxy,
- $R^3$ représente un groupe méthoxy,
- $R^4$ représente un groupe méthyle,
- et X représente un substituant oxo (=O).

**5.** Composé conforme à la revendication 1, dans lequel

- $R^1$ représente un groupe acétyle $-C(O)CH_3$,
- $R^2$ représente un groupe méthoxy,
- $R^3$ représente un groupe acétoxy,
- $R^4$ représente un groupe méthyle,
- et X représente un substituant oxo (=O).

**6.** Composition pharmaceutique comprenant, en une quantité efficace, un composé conforme à l'une des revendications 1 à 5, ainsi qu'un excipient pharmacologiquement admissible.

**7.** Composé conforme à l'une des revendications 1 à 5, ou composition pharmaceutique conforme à la revendication 6, pour utilisation dans la production d'un effet antiprogestatif chez une patiente.

**8.** Composé conforme à l'une des revendications 1 à 5, ou composition pharmaceutique conforme à la revendication 6, pour utilisation

a) dans l'induction de la menstruation,
b) dans le traitement d'une endométriose,
c) dans le traitement d'une dysménorrhée,
d) dans le traitement d'une tumeur dépendante d'une hormone endocrinienne,
e) dans le traitement d'un méningiome,

f) dans le traitement d'un fibrome utérin,
g) dans l'inhibition de la prolifération de l'endomètre dans l'utérus,
h) ou dans l'induction du travail chez une patiente.

**9.** Utilisation d'un composé conforme à l'une des revendications 1 à 5, ou d'une composition pharmaceutique conforme à la revendication 6, dans la préparation d'un agent contraceptif.

**10.** Utilisation d'un composé conforme à l'une des revendications 1 à 5, ou d'une composition pharmaceutique conforme à la revendication 6, dans la préparation d'un agent contraceptif post-coïtal.

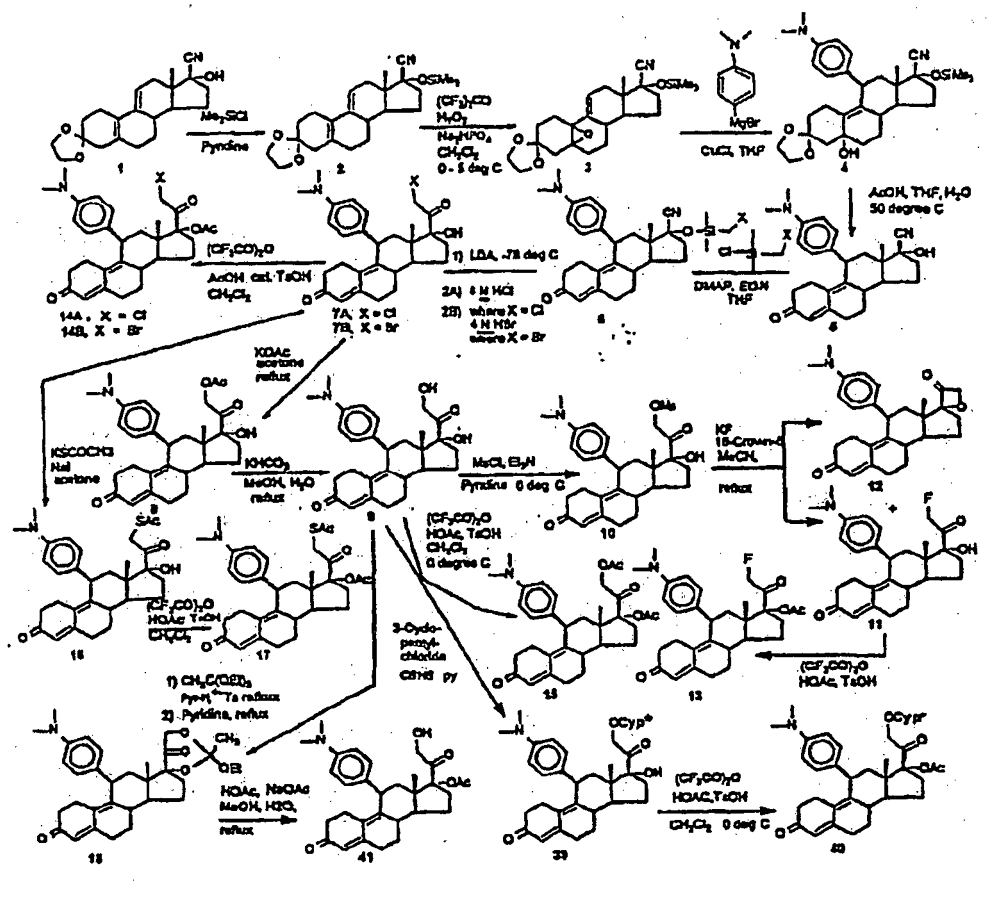
Pyridine
CuCl, THF
AcOH, THF, H2O
50 degree C
DMAP, Et3N
THF
1) LDA, -78 deg C
KOAc
acetone
reflux
KSCOCH3
NaI
acetone
KHCO3
MeOH, H2O
reflux
MsCl, Et3N
Pyridine 0 deg C
KF
18-Crown-6
MeCN
reflux
(CF3CO)2O
HOAc, TsOH
CH2Cl2
0 degree C
3-Cyclopentyl-chloride
2) Pyridine, reflux
HOAc, NaOAc
MeOH, H2O,
reflux

FIGURE 1

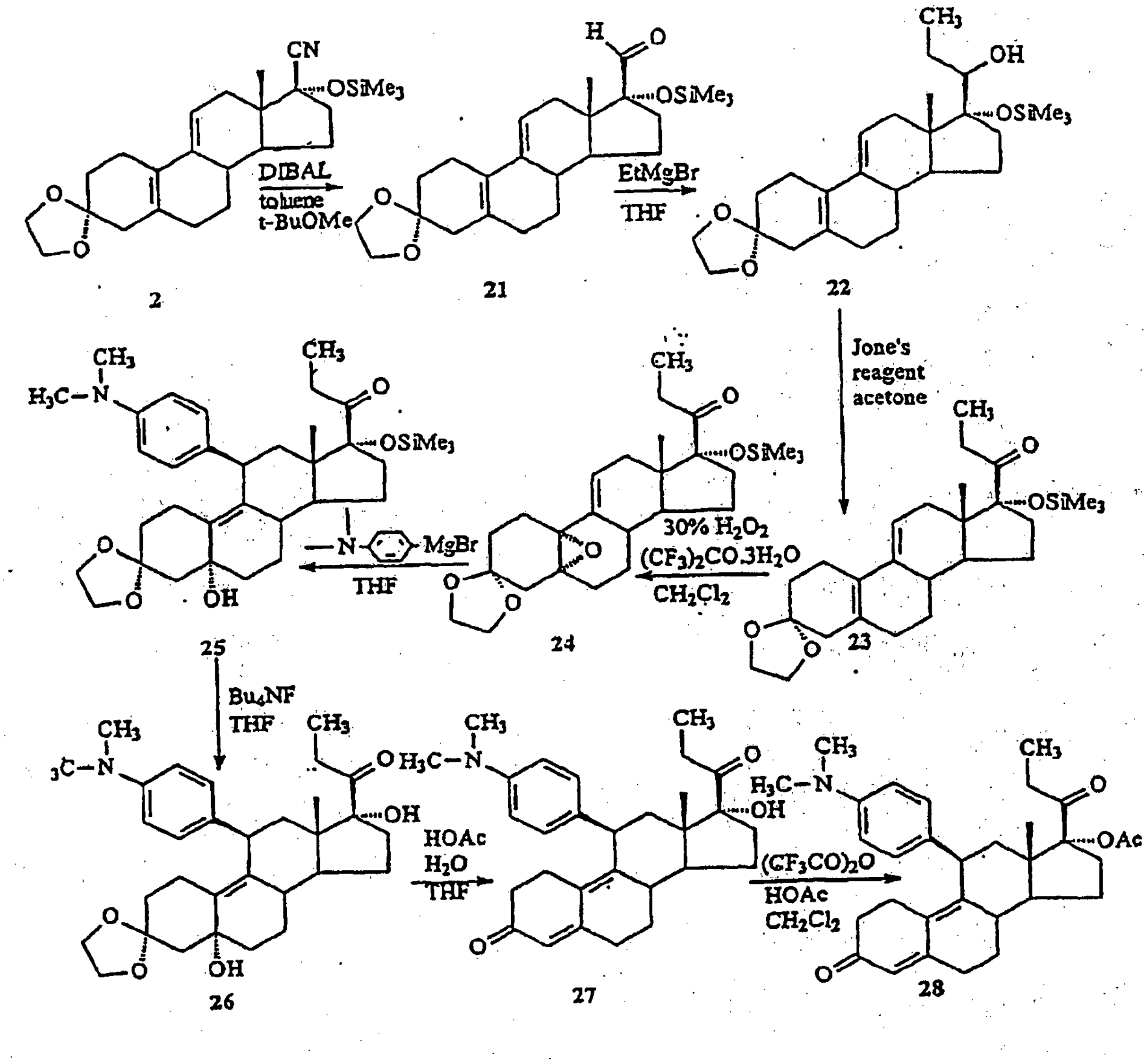


2 21 22 23 24 25 26 27 28

FIGURE 2

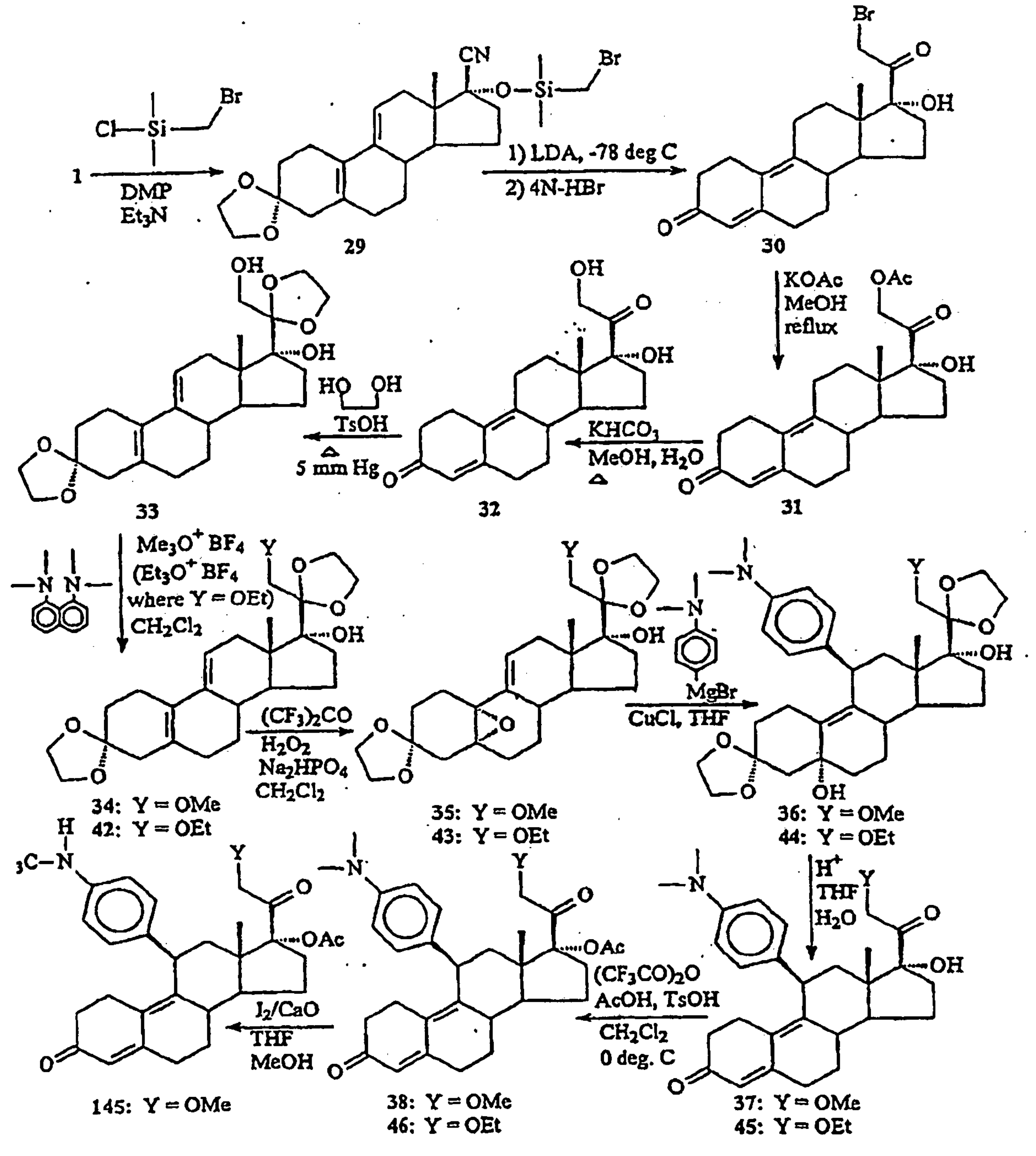


1

29

30

31

32

33

34: Y = OMe
42: Y = OEt

35: Y = OMe
43: Y = OEt

36: Y = OMe
44: Y = OEt

37: Y = OMe
45: Y = OEt

38: Y = OMe
46: Y = OEt

145: Y = OMe

FIGURE 3

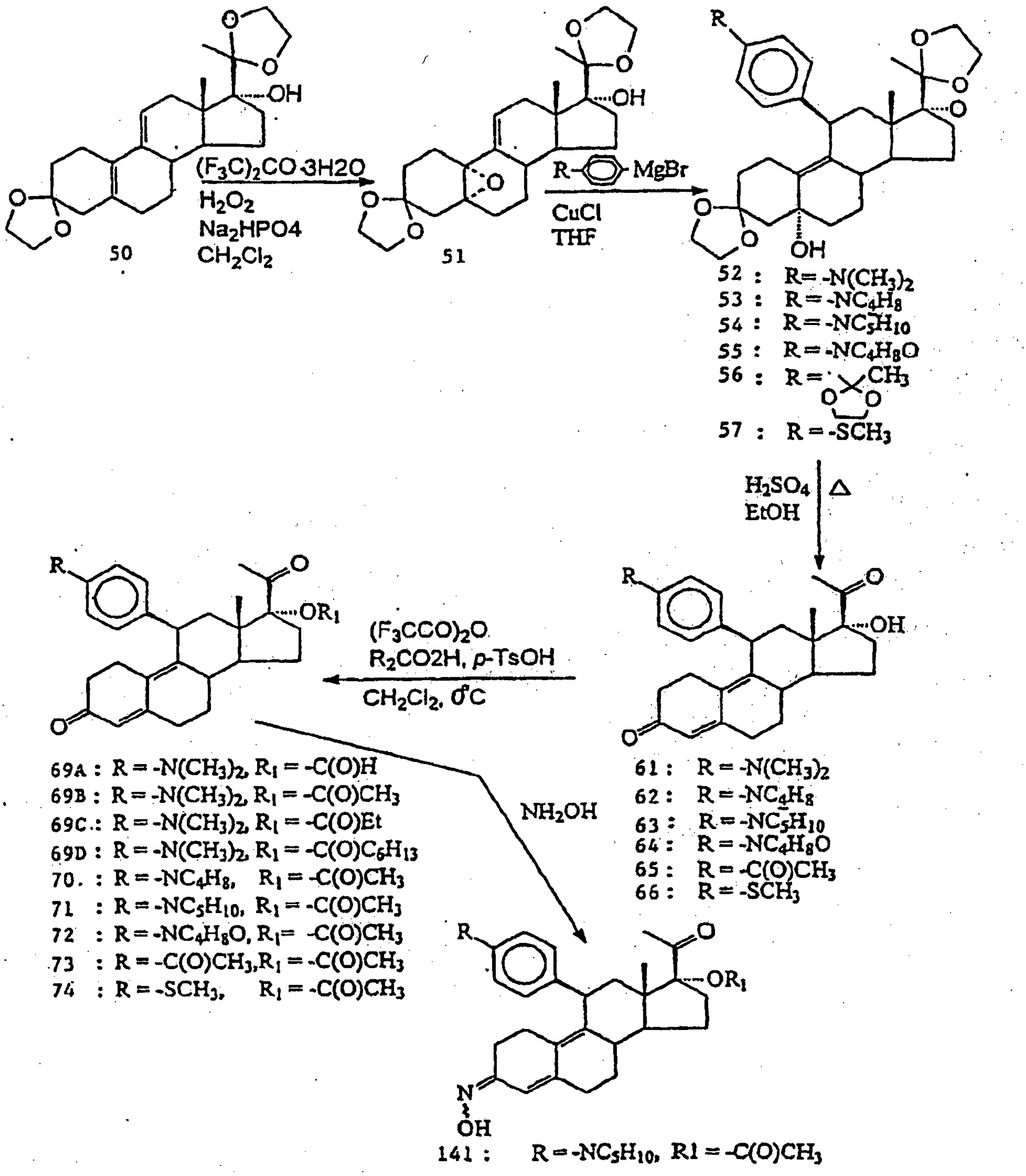


50

51

52 : R = $-N(CH_3)_2$
53 : R = $-NC_4H_8$
54 : R = $-NC_5H_{10}$
55 : R = $-NC_4H_8O$
56 : R =
57 : R = $-SCH_3$

61 : R = $-N(CH_3)_2$
62 : R = $-NC_4H_8$
63 : R = $-NC_5H_{10}$
64 : R = $-NC_4H_8O$
65 : R = $-C(O)CH_3$
66 : R = $-SCH_3$

69A : R = $-N(CH_3)_2$, $R_1$ = -C(O)H
69B : R = $-N(CH_3)_2$, $R_1$ = $-C(O)CH_3$
69C : R = $-N(CH_3)_2$, $R_1$ = -C(O)Et
69D : R = $-N(CH_3)_2$, $R_1$ = $-C(O)C_6H_{13}$
70 : R = $-NC_4H_8$, $R_1$ = $-C(O)CH_3$
71 : R = $-NC_5H_{10}$, $R_1$ = $-C(O)CH_3$
72 : R = $-NC_4H_8O$, $R_1$ = $-C(O)CH_3$
73 : R = $-C(O)CH_3$, $R_1$ = $-C(O)CH_3$
74 : R = $-SCH_3$, $R_1$ = $-C(O)CH_3$

141 : R = $-NC_5H_{10}$, R1 = $-C(O)CH_3$

Figure 4

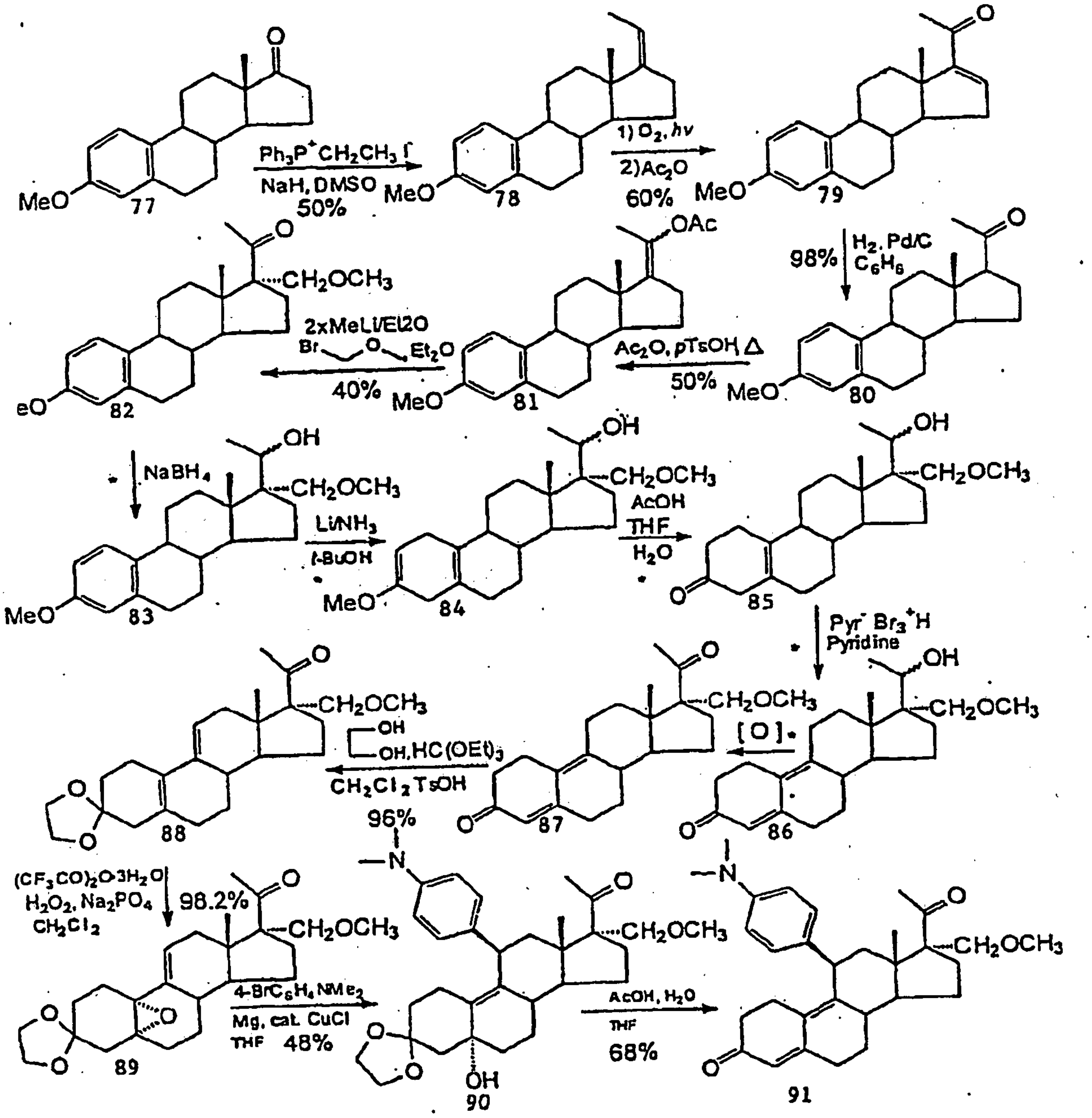


* Yield from 83 to 86is 37%

Figure 5

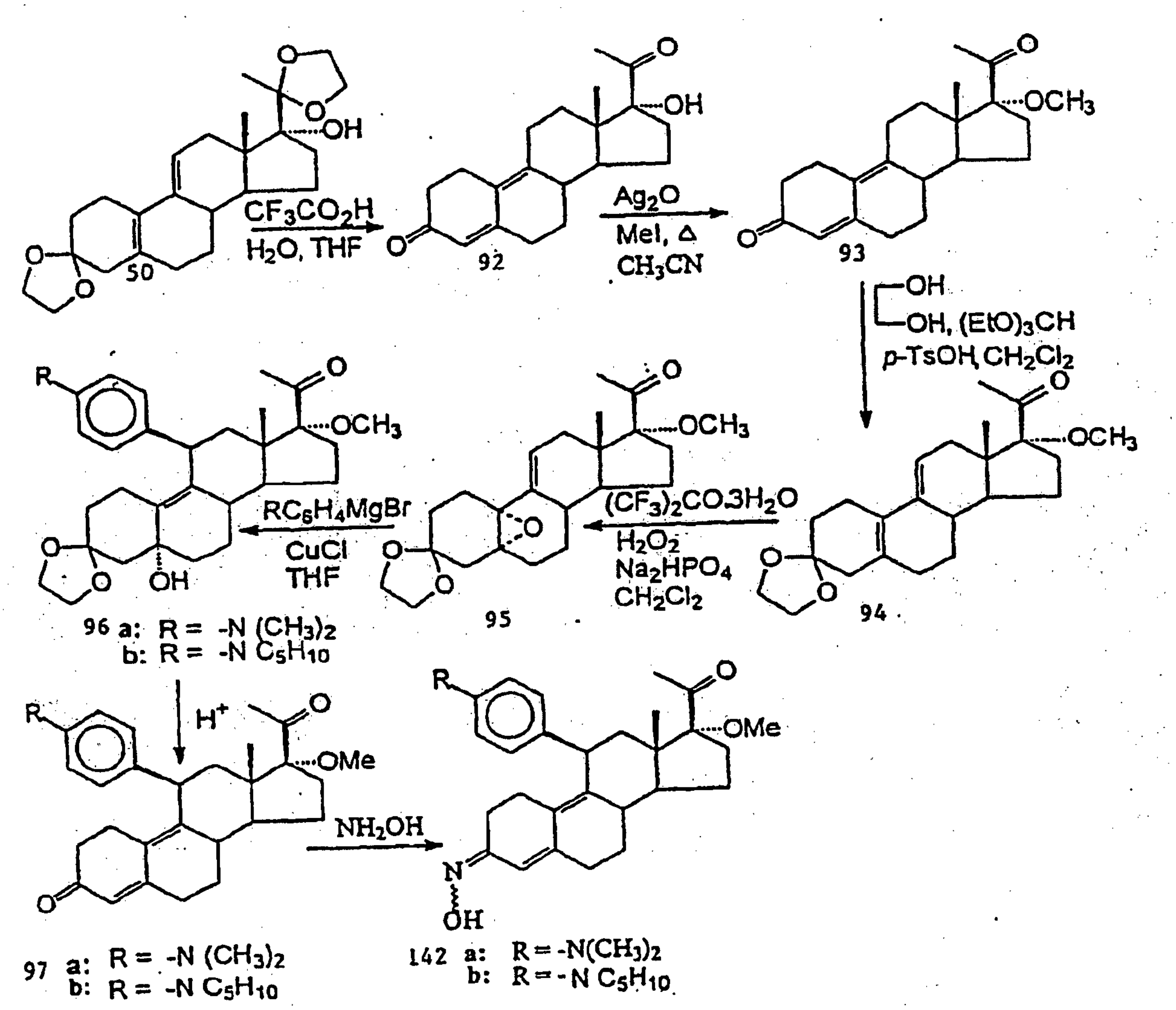
50
$CF_3CO_2H$
$H_2O$, THF
92
$Ag_2O$
MeI, Δ
$CH_3CN$
93
OH
OH, $(EtO)_3CH$
p-TsOH, $CH_2Cl_2$
94
$(CF_3)_2CO.3H_2O$
$H_2O_2$
$Na_2HPO_4$
$CH_2Cl_2$
95
$RC_6H_4MgBr$
CuCl
THF
96 a: R = -N $(CH_3)_2$
b: R = -N $C_5H_{10}$
$H^+$
$NH_2OH$
97 a: R = -N $(CH_3)_2$
b: R = -N $C_5H_{10}$
142 a: R = -N$(CH_3)_2$
b: R = -N $C_5H_{10}$

Figure 6

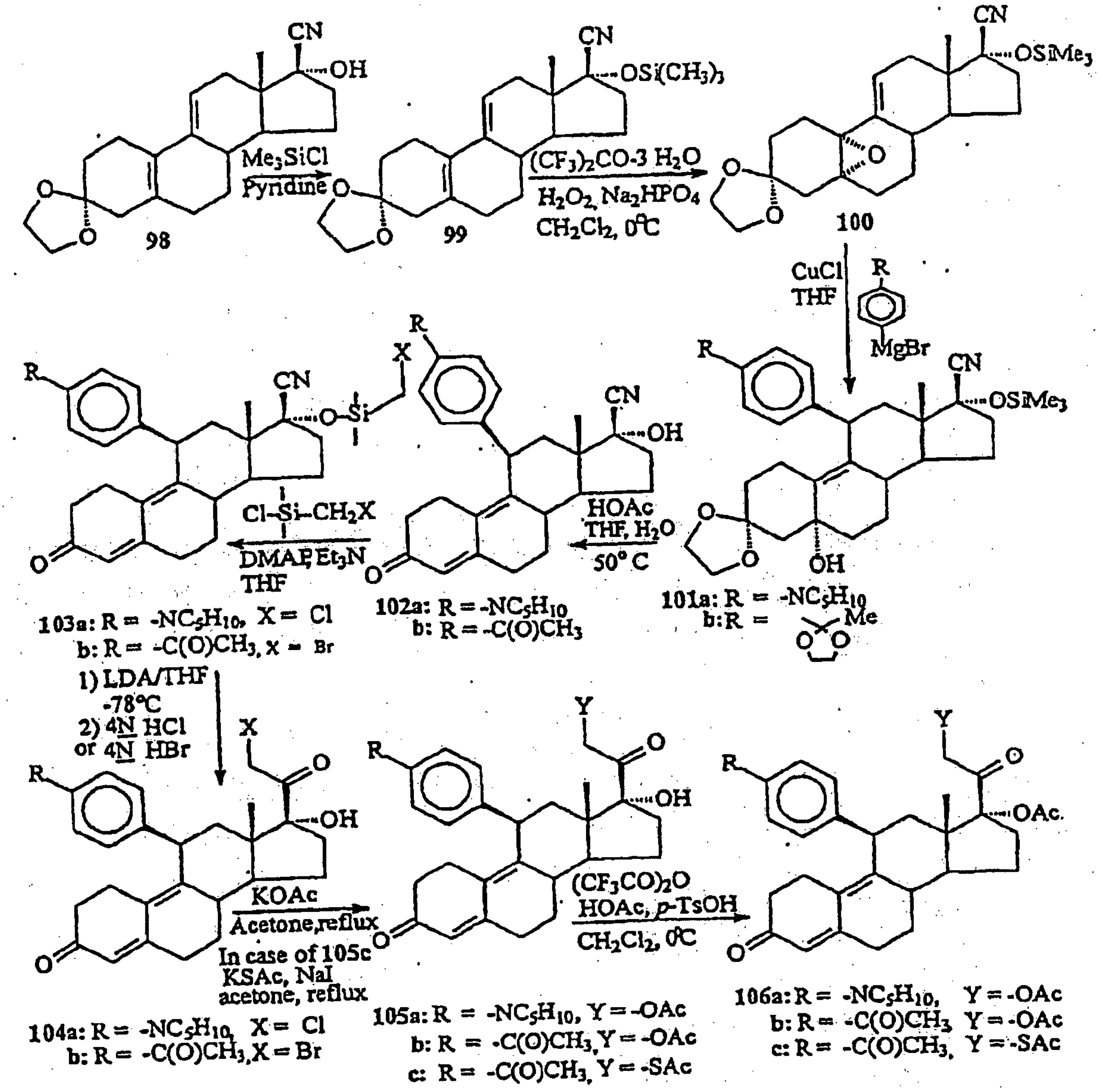
Me$_3$SiCl
Pyridine
98
99
($CF_3$)$_2$CO·3 $H_2O$
$H_2O_2$, $Na_2HPO_4$
$CH_2Cl_2$, 0°C
100
CuCl
THF
103a: R = -$NC_5H_{10}$, X = Cl
b: R = -C(O)$CH_3$, X = Br
Cl-Si-$CH_2$X
DMAP, $Et_3$N
THF
102a: R = -$NC_5H_{10}$
b: R = -C(O)$CH_3$
HOAc
THF, $H_2O$
50° C
101a: R = -$NC_5H_{10}$
b: R =
1) LDA/THF
-78°C
2) 4N HCl
or 4N HBr
KOAc
Acetone, reflux
In case of 105c
KSAc, NaI
acetone, reflux
104a: R = -$NC_5H_{10}$, X = Cl
b: R = -C(O)$CH_3$, X = Br
105a: R = -$NC_5H_{10}$, Y = -OAc
b: R = -C(O)$CH_3$, Y = -OAc
c: R = -C(O)$CH_3$, Y = -SAc
($CF_3$CO)$_2$O
HOAc, p-TsOH
$CH_2Cl_2$, 0°C
106a: R = -$NC_5H_{10}$, Y = -OAc
b: R = -C(O)$CH_3$, Y = -OAc
c: R = -C(O)$CH_3$, Y = -SAc

Figure 7

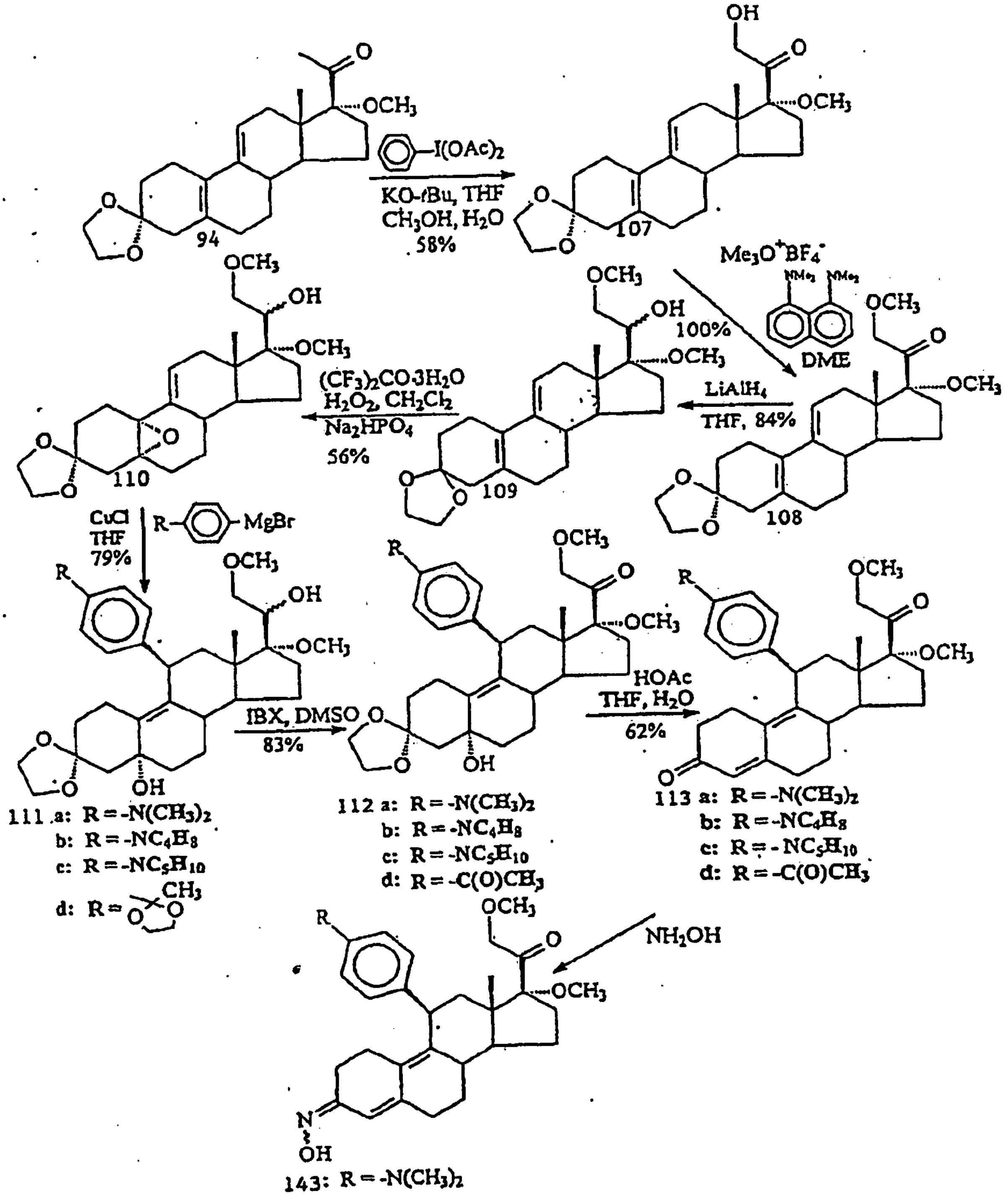


94

107

108

109

110

111 a: R = $-N(CH_3)_2$
b: R = $-NC_4H_8$
c: R = $-NC_5H_{10}$
d: R =

112 a: R = $-N(CH_3)_2$
b: R = $-NC_4H_8$
c: R = $-NC_5H_{10}$
d: R = $-C(O)CH_3$

113 a: R = $-N(CH_3)_2$
b: R = $-NC_4H_8$
c: R = $-NC_5H_{10}$
d: R = $-C(O)CH_3$

143: R = $-N(CH_3)_2$

Figure 8

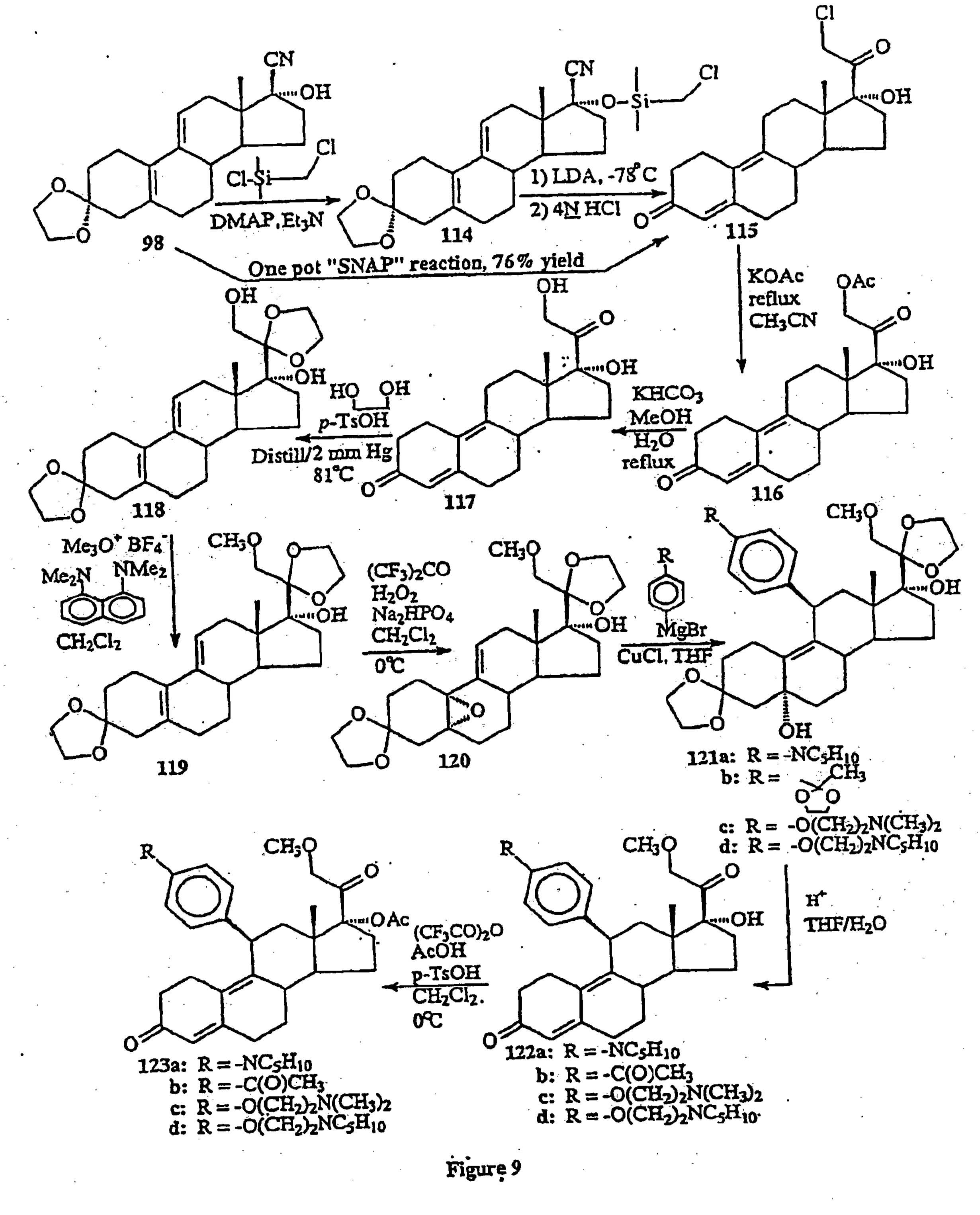


98

114

115

116

117

118

119

120

121a: R = -$NC_5H_{10}$
b: R =
c: R = -$O(CH_2)_2N(CH_3)_2$
d: R = -$O(CH_2)_2NC_5H_{10}$

122a: R = -$NC_5H_{10}$
b: R = -$C(O)CH_3$
c: R = -$O(CH_2)_2N(CH_3)_2$
d: R = -$O(CH_2)_2NC_5H_{10}$

123a: R = -$NC_5H_{10}$
b: R = -$C(O)CH_3$
c: R = -$O(CH_2)_2N(CH_3)_2$
d: R = -$O(CH_2)_2NC_5H_{10}$

Figure 9

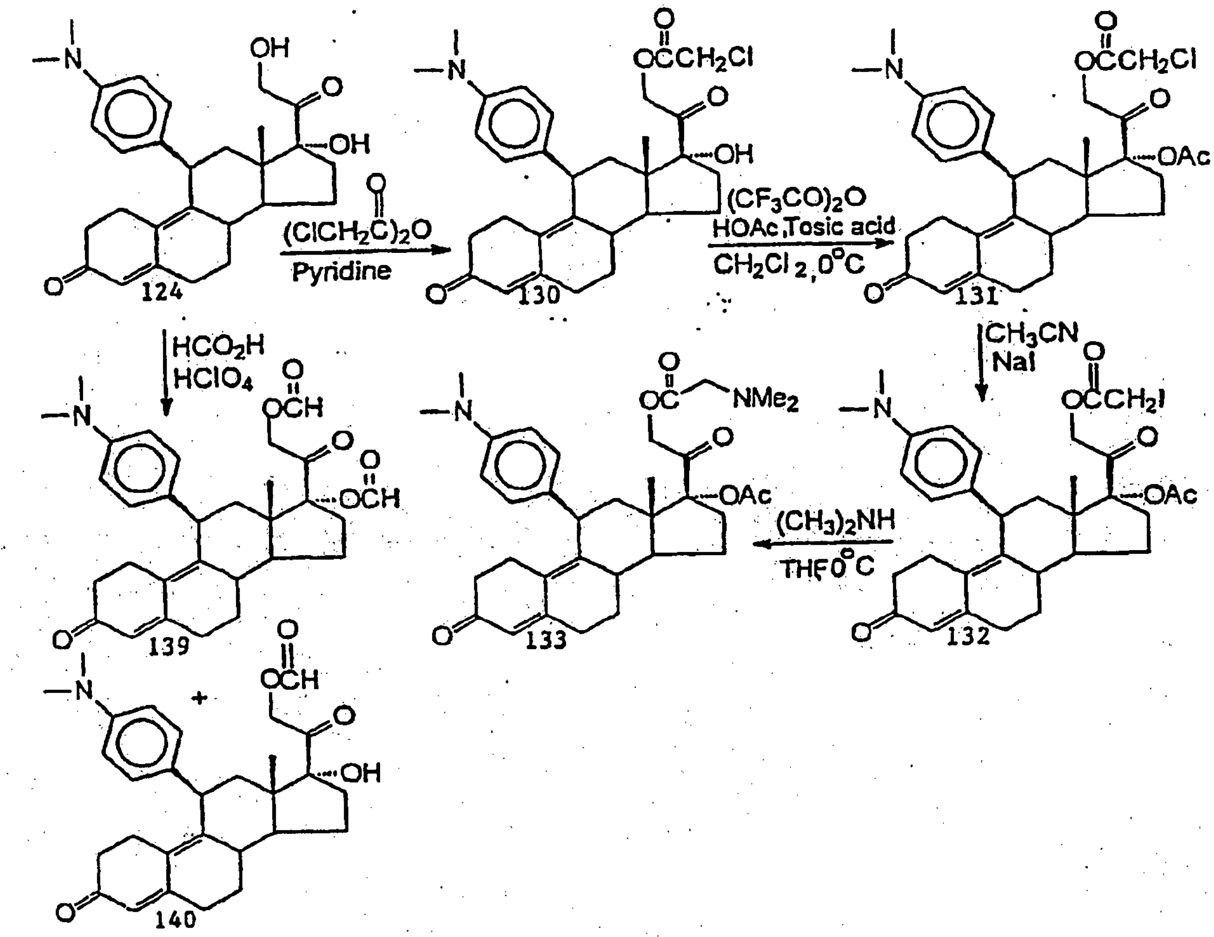


124 130 131 132 133 139 140

Figure 10

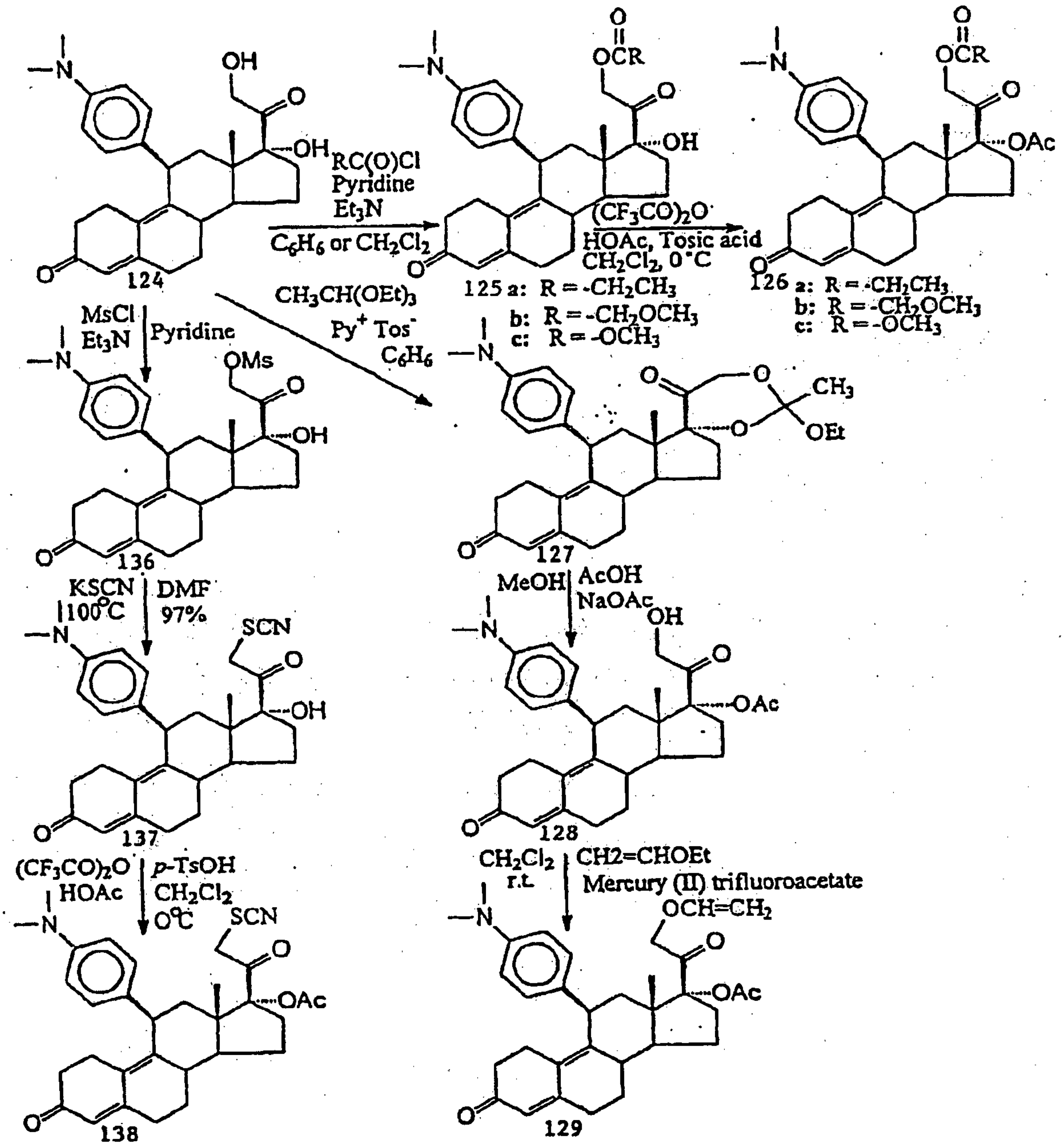


124

125 a: R = $-CH_2CH_3$
b: R = $-CH_2OCH_3$
c: R = $-OCH_3$

126 a: R = $-CH_2CH_3$
b: R = $-CH_2OCH_3$
c: R = $-OCH_3$

127

128

129

136

137

138

Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 8912448 A
- US 4092693 A, Livingston, D.A.
- US 4977255 A, Livingston, D.A.


### Non-patent literature cited in the description


- **G. TEUTSCH.** Adrenal Steroid Antagonism. Walter de Gruyter and Co, 1984, 43-75
- **NIEMAN et al.** *J. Clin, Endocrinology Metab.,* 1985, vol. 61, 536
- **HORWITZ.** *Endocrinology,* 1985, vol. 116, 2236
- **HEIKINHEIMO et al.** *J. Steroid Biochem.,* 1987, vol. 26, 279
- **KETTEL, L.M. et al.** *Fertil Steril,* vol. 56, 402-407
- **MURPHY, A.A. et al.** *Fertil Steril,* vol. 6, 3761-766
- **GROW, D.R et al.** *J. Clin. Endocrinol. Metab.,* vol. 81, 1933-1939
- **MURPHY, A.A. et al.** *J. Clin. Endocrinol. Metab.,* vol. 76, 513-517
- **BROGDEN, R.N. et al.** *Drugs,* vol. 45, 384-409
- **POISSON, M. et al.** *J. Neurooncol.,* vol. 1, 179-189
- **CARROLL, R.S. et al.** *Cancer Res.,* vol. 53, 1312-1316
- **MAHAJAN, D.K. ; LONDON, S.N.** *Fertil Steril,* 1997, vol. 68, 967-976
- **ROCHEFORT, H.** *Trends in Pharmacol. Sci.,* vol. 8, 126-128
- **HORWITZ, K.B.** *Endocr. Rev.,* 1992, vol. 13, 146-163
- **WOOD, A.J.J.** *N. engl. J. Med.,* 1993, vol. 329, 404-412
- **ULMANN, A. et al.** *Sci. Amer.,* 1990, vol. 262, 42-48
- **REEL, J.R. et al.** *Contraception,* 1998, vol. 58, 129-136
- **MORIARTY, R.M. et al.** *J. Chem. Soc. chem. Commun.,* 1981, 641-642
- **VELERIO et al.** *Steroids,* 1995, vol. 60, 268-271
- **LIVINGSTON, D.A. et al.** *J. Am. Chem. Soc.,* 1990, vol. 112, 6449-6450
- **LIVINGSTON, D.A.** *Adv.Med. Chem.,* 1992, vol. 1, 137-174
- **CARRUTHERS, N.I. et al.** *J. Org. Chem.,* 1992, vol. 57, 961-965
- **FINCH et al.** *J. Org. Chem.,* 1975, vol. 40, 206-215
- **NUMAZAWA, M. ; NAGAOKA, M.** *J. Chem. Soc. Commun.,* 1983, 127-128
- **NUMAZAWA, M ; NAGAOKA, M.** *J. Org. Chem.,* 1985, vol. 50, 81-84
- **GLAZIER, E.R.** *J. Org. Chem.,* 1962, vol. 27, 4397-4393
- **WIECHERT, R. ; NEEF, G.** *J. Steroid Biochem.,* 1987, vol. 27, 851-858
- **YUX'EV, Y.K. et al.** *Izvest. Akad. Nauk. S.S.S.R., Otdel Khim Nauk,* 1951, vol. 45, 166-171
- **WOLFE, J.P ; BUCHWALD, S.L.** *J. Org. Chem.,* 1997, vol. 62, 6066-6068
- **VERADRO, G. et al.** *Synthesis,* 1991, 447-450
- **JONES, D.H.** *J. Chem. Soc. (C),* 1971, 132-137
- **DETTY et al.** *J. Am. Chem. Soc.,* 1983, vol. 105, 875-882
- **RAO, P.N et al.** *Steroids,* 1998, vol. 63, 523-550
- **TEUTSCH, G ; BELANGER, A.** *Tetrahedron Lett.,* 1979, 2051-2054
- **DESS, D.B ; MARTIN, J.C.** *J. Org. Chem.,* 1983, vol. 48, 4155-4156
- **FRIGERIO, M ; SANTAGOSTINO, M.** *Tetrahedron Letters,* 1994, vol. 35, 8019-8022
- **FRIGERIO, M, et al.** *J. Org. Chem.,* vol. 60, 7272-7276
- **DETTY, M.R et al.** *J. Am. Chem. Soc.,* 1983, vol. 105, 875-882
- **RAO, P.N. et al.** *Steroids,* 1998, vol. 63, 523-530
- **MORIARTY et al.** *J. Chem. Soc., Chem. Commun.,* 1981, 641-642
- **BLISS, CL.** *The Statistics of Bioassay,* 1952
- **HOLLISTER, C.** *Nucleic Acids Research,* 1988, vol. 16, 1873-1875